# EUROPEAN PATENT SPECIFICATION

(11) **EP 4 111 966 B1**
(45) Date of publication and mention of the grant of the patent: **26.07.2023**
(21) Application number: 21202460.8
(22) Date of filing: 13.10.2021
(51) Int. Cl.: A61B 5/1455, A61B 5/16, G06F 3/01, G16H 20/70, G16H 50/20, A61B 5/055, A61B 5/11, A61B 5/12, G06V 40/20

(54) **DIGITAL CONTENT-BASED DEVICE FOR PROVIDING THERAPEUTICS INFORMATION**
AUF DIGITALEM INHALT BASIERENDE VORRICHTUNG ZUR BEREITSTELLUNG VON THERAPEUTISCHEN INFORMATIONEN
DISPOSITIF BASÉ SUR CONTENU NUMÉRIQUE POUR LA FOURNITURE D'INFORMATIONS THÉRAPEUTIQUES

(30) Priority: 30.06.2021 KR 20210085714
(43) Date of publication of application: 04.01.2023
(73) Proprietor: Actibrain Bio, Inc., Seoul 06112 (KR)
(72) Inventor: KIM, Sung Yeun, 06112 SEOUL (KR)
(74) Representative: ZBM Patents - Zea, Barlocci & Markvardsen

(56) References cited:
- KR-A- 20200 117 089
- KR-B1- 102 241 759
- US-A1- 2014 303 508

## Description

### BACKGROUND ART

### Technical Field

The present invention relates to a digital content-based device for providing therapeutics information and a method thereof. More particularly the present invention relates to a device for providing digital therapeutics and a method thereof by collecting signals related to a brain of a user in a state of activity and determining a brain state of the user based thereon.

### Description of the Related Art

The brain changes a neural circuit newly depending on people's behaviours and circumstances and generates new functions or cells resulting in growing up. Further, the function of brain areas may be changed according to what people thinks.

The hippocampus in the brain areas is involved in learning and memory and performs neurogenesis that generates new neurons, this neurogenesis occurring the most actively in the hippocampus. The next most active brain area next to the hippocampus in the neurogenesis is an olfactory related area. When taking a new smell, the brain generates new neurons to distinguish the smell.

However, all nerves fibers including brain cells become dead as time goes on rather than alive for a long-time. Since the cell death means aging or degeneration of cells, cell generation and neurodevelopment should be activated more than the cell death. Thus, in order to activate the nerve fibers, particularly brain cells, it is very crucial to find out factors affecting the rate of neurogenesis and the life span of neurons. When activation means to activate the brain cell generation, neurogenesis, neurogenesis rate and neuron life span, prior-used methods to activate the brain include exercise, foods, stimulants, music, meditation and the like. However, recently neurologists found out the aforementioned factors, these factors adopted to the brain activation to develop methods for improving brain functions.

In the meantime, when testing or diagnosing brain functions, used are various methods of computer tomography (CT), magnetic resonance imaging (MRI), proton emission tomography (PET), electroencephalograph (EEG), magnetoencephalography (MEG), functional magnetic resonance imaging (FMRI) and the like.

### Prior Art documents

### Patent Document

(Patent Document 1) Korean Patent No. 10-1754291 (July 06, 2017)
(Patent Document 2) Korean Patent Application Publication No. 10-2016-0058812 (May 25, 2016)
(Patent Document 3) Korean Patent No. 10-1768393 (August 17, 2017)
(Patent Document 4) Korean Patent No. 10-1295187 (August 9, 2013)
(Patent Document 5) Korean Patent No. 10-2241759 (April 20, 2021)

### SUMMARY

The present invention aims to provide a digital content-based device for providing therapeutics information so as to solve the aforementioned drawbacks of the prior art.

Particularly, the present invention aims to provide a device for providing digital therapeutics cur by collecting signals related to the brain of a user in a state of activity and determining a brain state of the user.

Further, the present invention aims to provide a device for providing digital therapeutics by performing stimulation on a brain of a user to obtain fNIRS data, extracting an activation area in a plurality of brain areas using the obtained fNIRS data, determining a brain state of the user based on the brain activation area and providing a determined content under an XR (extended reality) environment, allowing the user to perform a mission corresponding to the content.

Further, the present invention aims to provide the device for determining providing digital therapeutics by additionally extracting a brain activation area with respect to fNIRS data of the user who performed the mission, determining a brain state based on the additionally obtained brain activation area, and then determining and providing information related to amelioration of the brain state of the user.

Further, the present invention aims to provide a user with an AI based brain analysis technique capable of predicting an asymptomatic disease in advance and of early diagnosis.

Further, the present invention aims to provide a user with a brain function measurement technique which measures functions of a brain noninvasively in a way of mapping an average of time series blocks of the signal of channel position dependent fNIRS on a head, based on brain activation data imaged following measuring the brain in a state of activity.

Further, the present invention aims to provide a user with a brain activation area, a state cognitive algorithm and a data analysis visualization technique.

Further, the present invention aims to provide a user with a stimulation technique for obtaining brain activation data.

Further, the present invention aims to provide a user with a Hyper-scanning technique which measures changes in the brain of several persons and Inter-brain synchrony. Further, the present invention aims to provide a user with a determination technique of a brain activation state.

Further, the present invention aims to provide a user with a determination technique of a brain activation using body information (e.g., genetic information, gait pattern information, stress information, EGG change information, a sleep state and attention change information, oxygen saturation change information, and the like).

Further, the present invention aims to provide a user with a determination technique of a brain activation state using imaging scan information (e.g., MRI, PET, CT, fMRI, X-ray and the like).

Further, the present invention aims to provide a user with a technique for predicting, diagnosing and treating a disease, based on processing of a correlation of a plurality of data.

Further, the present invention aims to provide a user with a technique for obtaining data capable of used, as a biomarker in real time and analyzing the same.

Further, the present invention aims to provide a user with a technique for preventing problems related to dementia, MCI, Parkinson's disease, depression, cerebral stroke, Epilepsy, brain cancer and developmental disabilities, managing, diagnosing and treating those diseases.

Further, the present invention aims to provide a user a technique for providing feedback to the user following measuring brain functions, based on a metaverse environment. Meanwhile, technical aims to be achieved in the present invention are not limited to the aforementioned objects, and other not-mentioned technical aims will be obviously understood by those skilled in the art from the description below.

### Advantageous effects

According to the present invention, there is provided a digital content-based device for providing therapeutics information according to claim 1.

Particularly, the present invention is capable of providing the device for providing digital therapeutics by collecting signals related to a brain of a user in a state of activity and determining a brain state of the user based thereon.

Further, the present invention is capable of providing the device for providing digital therapeutics by performing stimulation on the brain of the user to obtain fNIRS data, extracting an activation area in a plurality of brain areas using the obtained fNIRS data, determining a brain state of the user based on the brain activation area and providing a determined content under an XR (extended reality) environment, allowing the user to perform a mission corresponding to the content.

Further, the present invention is capable of providing the device for determining providing digital therapeutics by additionally extracting a brain activation area with respect to fNIRS data of the user who performed the mission, determining a brain state based on the additionally obtained brain activation area, and then determining and providing information related to amelioration of the brain state of the user.

Further, the present invention is capable of providing the user with an AI based brain analysis technique which allows prediction of an asymptomatic disease in advance and early diagnosis.

Further, the present invention is capable of providing the user with a brain function measurement technique which measures functions of the brain noninvasively in a way of mapping an average of time series blocks of the signal of channel position dependent fNIRS on a head, based on brain activation data imaged following measuring the brain in a state of activity.

Further, the present invention is capable of providing the user with a brain activation area, a state cognitive algorithm and a data analysis visualization technique.

Further, the present invention is capable of providing the user with a stimulation technique for obtaining brain activation data.

Further, the present invention is capable of providing the user with a Hyper-scanning technique which measures changes in the brain of several persons and Inter-brain synchrony.

Further, the present invention is capable of providing a user with a determination technique of a brain activation state.

Further, the present invention is capable of providing the user with a determination technique of the brain activation state using body information (e.g., genetic information, gait pattern information, stress information, EGG change information, a sleep state and attention change information, oxygen saturation change information, and the like).

Further, the present invention aims to provide a user with a determination technique of a brain activation state using imaging scan information (e.g., MRI, PET, CT, fMRI, X-ray and the like).

Further, the present invention is capable of providing the user with a technique for predicting, diagnosing and treating a disease, based on a correlation of a plurality of data.

Further, the present invention is capable of providing the user with a technique for obtaining data capable of used, as a biomarker in real time and analyzing the same.

Further the present invention is capable of providing the user with a technique for preventing problems related to dementia, MCI, Parkinson's disease, depression, cerebral stroke, Epilepsy, brain cancer and developmental disabilities, managing, diagnosing and treating those diseases.

Further, the present invention is capable of providing the user a technique for providing feedback to the user following measuring brain functions, based on a metaverse environment.

Meanwhile, advantageous effects obtained from the present invention are not limited to the aforementioned effects, and other not-mentioned effects will be obviously understood by those skilled in the art from the description below.

### DESCRIPTION OF THE DRAWINGS

The technical essence of the present disclosure will be more clearly understood from the following detailed description taken in conjunction with the accompanying drawings. Therefore, the present invention will not be interpreted to be limited to the drawings in which:
FIG. 1 is a block diagram of an AI based device for providing brain information in connection with the present invention.
FIG. 2 is a block diagram of a brain signal measurement portion that is a configuration element of the AI based device for providing brain information in connection with the present invention.
FIG. 3 is a view for explaining the operation of the brain signal measurement portion in connection with the present invention.
FIG. 4A and 4B are views for explaining fNIRS (functional near-infrared spectroscopy) to be adopted to the present invention.
FIG. 5 and FIG. 6 show one example in which signals measured by the brain signal measurement portion are visualized and displayed in connection with the present invention.
FIG. 7A to FIG. 7C show one example of each of the signals measured by the brain signal measurement portion, visualized information and processed information in connection with the present invention.
FIG. 8 is a block view of a brain signal stimulating portion that is a configuration element of the AI based device for providing brain information.
FIG. 9 is a block diagram of a user data collecting portion in connection with the present invention.
FIG. 10 is a block diagram of an imaging obtaining portion in connection with the present invention.
FIG. 11 is an exemplary view showing analysis data in an SMA by using a z-score analysis method according to the embodiment of the present invention.
FIG. 12 is a view for explaining a multisensor biodata based model for providing activated XR digital therapeutics in connection with the present invention.
FIG. 13 and FIG. 14 show one example of each biodata obtained in connection with the present invention.
FIG. 15 is a view for explaining one example which adopts the digital content-based method and system for providing therapeutics information in connection with the present invention.
FIG. 16 shows one example of a flowchart for explaining the digital content-based method for providing therapeutics information in connection wth the present invention.
FIG. 17 and FIG. 18 show views for explaining a technique for predicting, diagnosing and treating a disease, based on processing of a correlation of a plurality of data in connection with the present invention.
FIG. 19A to 19C show one example for providing a content determined corresponding to the brain state of the user under an XR (Extended Reality) environment.
FIG. 20A to FIG. 20C show one example for that the user performs a mission corresponding to the content provided under the XR environment and monitors the brain state.

### DETAILED DESCRIPTION OF EMBODIMENTS

### Degenerative Brain Disease

The elderly population ratio (aged 65 or over) in Korea was 13.2% of the total population as of year 2019, however, as the aged population is increasing rapidly, it will be expected to reach 20% as of year 2025.

For the next 45 years, each proportion of a youth population and a working age population is expected to be decrease 3.7%p and 23.3%p, respectively and the elderly population ratio is expected to occupy 40.1 % in the total population in 2060.

It is expected that elder care expenses in Korea will increase continuously, reaching 2 times of those in US by 2050 and furthermore this will the highest in the world along with Japan in 2060.

A spectacular increase in the population with dementia to be foreseen in the super-aged society appears and the prevalence rate of dementia shows an increasing trend with age.

Accordingly, dementia is one of urgent problems to be overcome in the modern world that will be entered into the super-aged society before long. The number of the world's dementia prevalence is 4,600 which is greater than the population in Spain, showing a two-fold increase every 20 years and thus will increase rapidly to 130,000,000 by 2050.

It is expected that the East Asian population with dementia will increase 193% by 2050 and the number of domestic patients with dementia is estimated to reach 2,710,000 by 2050.

Accordingly, national dementia care expenses increase resulting in causing social and economic burdens. The spectacular increase in patients with dementia causes an increase in national dementia care expenses. The national dementia care expenses approximated trillion won as of the year 2019 (about 0.7% of GDP) and will increase 2 times every 10 years. Thus, this is estimated to exceed 100 trillion won in 2050 (about 2.0% of GDP).

These economic expenses resulting from dementia are 2 times and 3 times of those for cardia disease and cancer, respectively. The worldwide social·economic expenses was 81.8 billion dollar as of the year 2015 and is expected to increase to 2 trillion dollars by 2030.

Domestic social economic losses approximate 2 trillion won per year, increasing 2 times or more during recent 4 years (Reference: Search Report of Korean Institute for health & Welfare Policy).

The worldwide aging population increases the incidence of degenerative brain diseases and social problems. Even though many studies on the treatment and prevention of degenerative brain diseases including dementia are being conducted actively, these are at a standstill. Currently, dementia diagnosis is available through a thorough medical checkup in big hospitals and university hospitals, resulting in problems in terms of psychological, geographical and economic accessibility.

The current screening rate of dementia is 45% (for reference, prevalence rates of 4 major types of cancers are 90% or more), non-invasive dementia diagnosis methods are proposed in various fields. However, there has been not any study on a diagnosis method of dementia on the basis of information gathered through real-time monitoring.

Further, a failure rate of developing a treatment agent for dementia approximates 9.6%, and solanezumab expected as a treatment agent targeting amyloid beta has failed to show promise in Phase III trials.

The reason why a success rate of developing a treatment agent of dementia does not reach 1% is that no onset mechanism was found clearly.

### Problems of Prior Diagnosis Technique

The current dementia diagnosis is made up through comprehensive determination on the basis of various tests including brain imaging, electroencephalography, blood test, cerebral spinal fluid examination, Neuropsychological test, etc.

In regard of expenses, the brain imaging is widely used because brain's form such as cerebral atrophy can be seen directly. This uses various kinds of imaging techniques are used including Magnetic Resonance Imaging (MRI), Single-Photon Emission Computed Tomography (SPECT), Positron Emission Tomography (PET), etc., the PET imaging scan helping early diagnosis.

The electroencephalography checks overall cognitive decline by examining memory, attention, visuospatial sense, calculation ability, etc. This is used for early diagnosing a disease, monitoring disease progression and determining efficacies of treatment drugs.

However, the most major drawback of this examination is that the accuracy rate of the examination is proportional to the progression rate for dementia, this meaning that the rate of misdiagnosis is high at very early stage showing no clear symptom.

Further, in a case of cerebral spinal fluid examination, biomarkers accumulated in the brain may be detected directly. However, there is a drawback that it is very painful and inconvenience to collect cerebral spinal fluid for a subject.

### Problems of Prior Art related to Child's Brain Development

As of the year 2019, each population with intellectual disabilities, autism and ADHA is 213,000, 29,000 and 2,060,000, respectively.

The intellectual disabilities and the autism are slow of treatment effects, while in a case of the ADHA, appropriate treatments during childhood avoids continuing this to adulthood. Thus, early diagnosis and treatment of the ADHA is required.

In order to measure a child's brain state with existing MRI, fMRI, CT, etc., it is required that the child hardly moves, differently from his/her developmental features. In a case of toddlers, it is essentially required to administer a sleeping dug and an anesthetic drug.

At this time, when a baby aged less than 2 years is exposed to the anesthetic drug several times, a risk of the occurrence of permanent learning impairment increases 2 times.

Further, in order to develop various kinds of new medicines for brain development, it is required to invest a lot of time and to collect biodata for a certain period of time.

There is a method to check the brain development through multilateral interaction but in a case of the existing product, hyperscanning for measuring a brain state multilaterally is not allowable and thus diversity of such a method for measuring the brain state is required. Accordingly, demands for developing products satisfying this requirement and mobile instruments for measuring the brain state are increasing.

### Objects of the Invention

Therefore, the present invention intends to contribute to solve social problems of neurologic diseases through building of a deep-learning dataset for early diagnosis and treatment of degenerative brain diseases increasing following aging population. That is, in order to solve aforementioned problems, required are techniques for Active MRI brain activity assessment of a person with dementia and for early diagnosis of dementia.

Since clinical symptoms are observed long before occurrence of deteriorations in brain structure and function, it is important to assess or diagnose dementia early through the measurement of brain's ability to function.

In order for Al's recognition and understanding of its own accord, it is important to obtain a large scale of AI learning data processed in a form that an AI SW is capable of understanding association between things, AI performance based on machine learning is determined according to utilization of a huge amount of data collected under various circumstances and it is also important that there is synergy between the data and the AI.

According to another object of the present invention, provided are, for a child, a multilateral wireless brain signal measurement device for measuring brain in real time and a brain development examination service.

In order to identify child's brain development state and to held child grow up healthy, the present invention is provided 1) to prevent and diagnose brain diseases such as ADHA, and 2) to provide experience value for cultivating child's attention in everyday life and learning.

Further, the present invention intends to provide a device capable of collecting measure results of brain states for a certain period of time and a solution providing a brain developing program which is, on the basis of the collected data, capable of predicting brain disease risk and is adoptable to everyday life and learning. According to another object of the present invention, in order to support fields of non-contact learning and emotional work, provided is a sensitivity cognitive/sharing AI (Human-centered AI) service utilizing the construction of a multimodal emotional data network and the N-dimensional emotion mapping.

The emotion cognitive technique adopts a technique which defines an event stream (user's behavior) utilizing ICT and intelligence quotients for social emotional cognition and emotion sharing through a non-contact vital reaction and which is capable of recognize basic emotion and social emotion in a space of the N dimensional emotion mapping.

Further, the non-contact vital reaction is capable of utilizing measurement of a biosignal based five senses by using a sensor camera, an AI speaker, etc. which are usable in everyday life.

Further, the present invention intends to provide and build a data network which is capable of extending the user's behavior event and non-contact vital reaction step by step from a unimodal typed data network to the multimodal data network through which being capable of mapping with the social emotion and is also capable of growing up and evolving through AI.

Further, the emotion AI service may provide a whole new level of human-centered AI service which overcomes unpredictable behavior or emotion of a learner or an emotional labor from a cognitive level to an intuitive level.

In order to achieve the aforementioned objects, the present invention intends to provide a device for providing digital therapeutics by collecting signals related with the brain in a state that the user is moving and determining the brain state of the user based thereon.

Further, the present invention intends to provide the device for providing digital therapeutics by performing stimulation on a brain of a user to obtain fNIRS data, extracting an activation area in a plurality of brain areas using the obtained fNIRS data, determining a brain state of the user based on the brain activation area and providing a determined content under an XR (extended reality) environment, allowing the user to perform a mission corresponding to the content.

Further, the present invention intends to provide the device for determining providing digital therapeutics by additionally extracting a brain activation area with respect to fNIRS data of the user who performed the mission, determining a brain state based on the additionally obtained brain activation area, and then determining and providing information related to amelioration of the brain state of the user.

Further, the present invention intends to provide a user with an AI based brain analysis technique capable of predicting an asymptomatic disease in advance and of early diagnosis.

Further, the present invention intends to provide the user with a brain function measurement technique which measures functions of a brain noninvasively in a way of mapping an average of time series blocks of the signal of channel position dependent fNIRS on a head, based on brain activation data imaged following measuring the brain in a state of activity.

Further, the present invention intends to provide the user with a brain activation area, a state cognitive algorithm and a data analysis visualization technique.

Further, the present invention intends to provide the user with a stimulation technique for obtaining brain activation data.

Further, the present invention intends to provide the user with a Hyper-scanning technique which measures changes in the brain of several persons and Inter-brain synchrony.

Further, the present invention intends to provide the user with a determination technique of a brain activation state.

Further, the present invention intends to provide the user with a determination technique of a brain activation using body information (e.g., genetic information, gait pattern information, stress information, EGG change information, a sleep state and attention change information, oxygen saturation change information, and the like).

Further, the present invention intends to provide the user with a determination technique of a brain activation state using imaging scan information (e.g., MRI, PET, CT, fMRI, X-ray and the like).

Further, the present invention intends to provide the user with a technique for predicting, diagnosing and treating a disease, based on a correlation of a plurality of data.

Further, the present invention intends to provide the user with a technique for obtaining data capable of used, as a biomarker in real time and analyzing the same.

Further, the present invention intends to provide the user with a technique for preventing problems related to dementia, MCI, Parkinson's disease, depression, cerebral stroke, Epilepsy, brain cancer and developmental disabilities, managing, diagnosing and treating those diseases.

Further, the present invention intends to provide the user a technique for providing feedback to the user following measuring brain functions, based on a metaverse environment.

Hereinafter, the AI based device for providing brain information will be described in detail with the accompanying drawings.

### AI based Device for Providing Brain Information

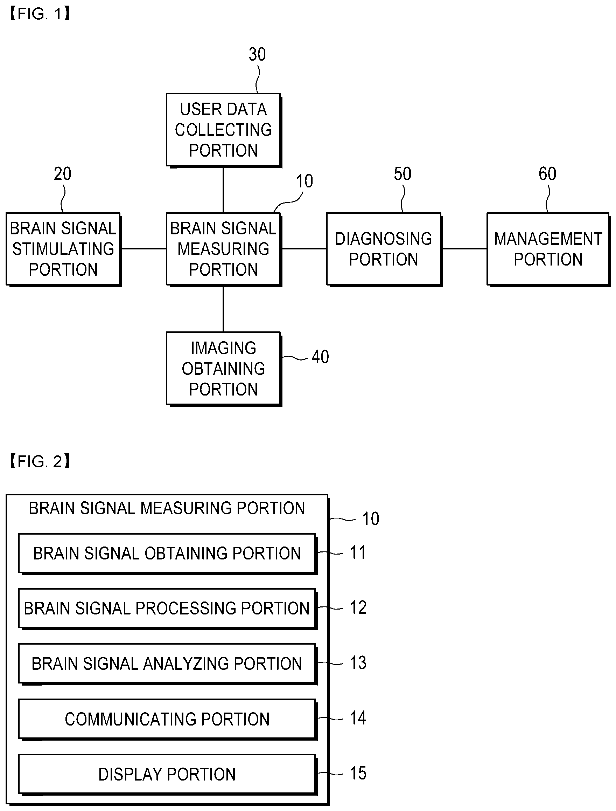

FIG. 1 is a block diagram showing an AI based device for providing brain information in connection with the present invention.

Referring to FIG. 1, the AI based device 1 for providing brain information according to the present invention may include a brain signal measuring portion 10, a brain signal stimulating portion 20, a user data collecting portion 30, an imaging obtaining portion 40, a diagnosing portion 50 and a management portion 60.

Firstly, the brain signal measuring portion 10 collects signals related to a brain of a user.

In particular, the brain signal measuring portion 10 according to the present invention irradiates a near-infrared ray to the user's brain and detects light penetrating the cerebral cortex of the brain, determines a level of oxygenation of hemoglobin in blood flow of the user's brain based on the detected light and extracts at least one brain activation area from a plurality of brain areas of the user based on the determined level of oxygenation of hemoglobin.

The present invention is characterized in that a signal collecting operation of the brain signal measuring portion 10 may be performed in a state that the user is moving. Moving on to the next, the brain signal stimulating portion 20 stimulates the user's brain for the signal collecting operation of the brain signal measuring portion 10.

Furthermore, the brain signal stimulating portion 20 stimulates the user's brain so as to ameliorate a brain state of the user according to controlling of the management portion.

Further, the user data collecting portion 30 collects body information related to the user. The body information related to the user may include the user's auditory information, gait information, stress information, electrocardiogram information, sleep information, attention information, emotional change information and the like.

Further, the imaging obtaining portion 40 collects medical imaging related to the user. The imaging obtaining portion 40 according to the present invention may use an MRI imaging scan obtaining portion that collects an MRI imaging scan related to the user, a CT imaging scan obtaining portion that collects a CT imaging scan related to the user, an fMRI imaging scan obtaining portion that collects an fMRI imaging scan related to the user and the like.

Further, the diagnosing portion 50 determines the brain state of the user based on the collected signals from the brain signal measuring portion 10.

The diagnosing portion 50 may determine a brain disease of the user. Target diseases may include dementia, Parkinson's disease, cerebral stroke, Epilepsy, brain cancer and developmental disabilities.

Lastly, the management portion 60 provides information for ameliorating the brain state of the user, corresponding to the brain state of the user determined by the diagnosing portion 50.

For example, when the user, by applying a preset reference, is suspected of at least one of dementia, Parkinson's disease, cerebral stroke, Epilepsy, brain cancer and developmental disabilities within a certain period of time, based on the brain state of the user, an operation of the brain signal stimulating portion 20 for stimulating the user's brain may be triggered for ameliorating the rain state of the user according to controlling of the management portion 60.

Hereinafter, respective configuration elements will be described in regard of specific structures and functions with reference to drawings.

### Brain signal measuring portion

FIG. 2 is a block diagram of a brain signal measuring portion that is a configuration element of the AI based device for providing brain information.

Referring to FIG. 2, the brain signal measuring portion 10 may include a brain signal obtaining portion 11, a brain signal processing portion 12, a brain signal analyzing portion 12, a communicating portion 14 and a display portion 15.

Firstly, the brain signal obtaining portion 11 irradiates a near-infrared ray to the user's brain and detects light penetrating the cerebral cortex of the brain.

Wherein, a signal collecting operation of the brain signal obtaining portion 11 is performed in a state that the user is moving.

Moving on to the next, the brain signal processing portion 12 determines a level of oxygenation of hemoglobin in blood flow of the user's brain based on the detected light. The brain signal processing portion 12 extracts an oxygenated hemoglobin (Oxy Hb) concentration and a deoxygenated hemoglobin (Deoxy Hb) concentration in blood flow of the user to determine a level of oxygenation of hemoglobin.

Further, the brain signal analyzing portion 13 extracts at least one brain activation area from a plurality of brain areas of the user based on the determined level of oxygenation of hemoglobin.

Wherein, in the at least one brain activation area, oxygen transferred by the Oxy Hb in blood flow is consumed to decrease the Oxy Hb concentration and to increase the Deoxy Hb concentration corresponding to the decreased Oxy Hb concentration.

The Oxy Hb concentration and the Deoxy Hb concentration have an optical property that changes in a visible-ray area and a near-infrared area and consequentially, the brain signal measuring portion 10 collects the signal through fNIRS (functional Near-infrared Spectroscopy).

Meanwhile, the brain signal analyzing portion 13 divides the brain area of the user into multiple areas and determines whether or not each of the divided brain areas is changed to the brain activation area by a predetermined time unit, or whether or not the brain activation area is changed to an inactivation area.

Based thereon, the diagnosing portion 50 may determine the brain state of the user using brain activation changes of the respective divided brain areas with reference to a predetermined period of time.

Further, the communicating portion 14 builds networks between the brain signal measuring portion 10 and other configuration elements (the brain signal stimulating portion 20, the user data collecting portion 30, the imaging obtaining portion 40, the diagnosing portion 50, the management portion 60, etc.) to support data communication.

Wherein, usable wireless communication techniques may include Wireless LAN (WLAN) (Wi-Fi), Wireless broadband (Wibro), World Interoperability for Microwave Access (Wimax), High Speed Downlink Packet Access (HSDPA) and the like.

Further, usable short range communication techniques may include Bluetooth, Radio Frequency Identification (RFID), Infrared Dara Association (IrDA), Ultra-Wideband (UWB), ZigBee and the like.

Further, the display portion 15 displays the brain activation change in each of the plurality of the divided brain areas according to the time.

The display portion 15 may include at least one of a Liquid Crystal Display (LCD), a Thin Film Transistor-Liquid Crystal Display (TFT LCD), an Organic Light-Emitting Diode (OLED), a flexible display and a 3D display.

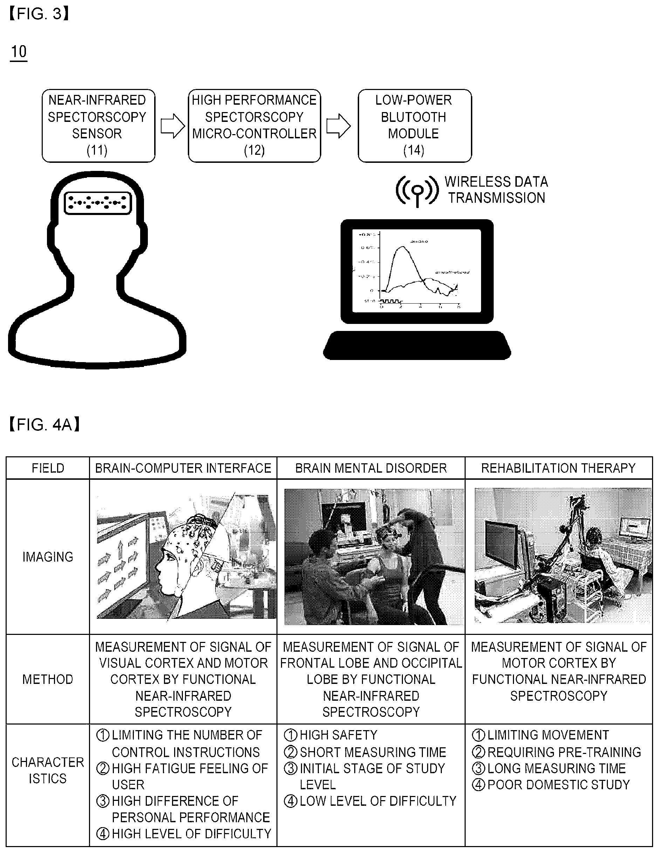

FIG. 3 is a view for explaining the operation of the brain signal measurement portion in connection with the present invention.

Referring to FIG. 3, the brain state of the user and a development state thereof are measured multilaterally in real time in a state that the user is moving. That is, a brain signal measuring device is provided which is capable of measuring brain signals of a multiuser so as to analyze the brain signal wirelessly in real time without any limit.

The brain signal measuring portion 10 according to the present invention may be a full cover type which covers the whole of the user's head, alternatively a front type which is adhered closely to a front part of the user's head.

Further, as considering circumstances particularly being used for a child, in a case of the brain signal measuring portion 10, a UI/UX custom design for providing experience values may be considered which adopts a child custom design and makes up a user friendly environment.

In the present invention, a wearable brain signal measuring technology may be adopted which is capable of being used for a long time by using Bluetooth low energy (BLE). Further, a flexible substrate is used for designing the brain signal measuring device for a child to fit baby's head shape, allowing weight minimization of the device. Further, used are a brain signal analyzing algorithm that is capable of measuring attention and brain development of the user in terms of physiology through brain activation analyzation, a data transmission algorithm that is capable of securing data reliability even if a channel state is changed, and a brain activation area cognitive algorithm.

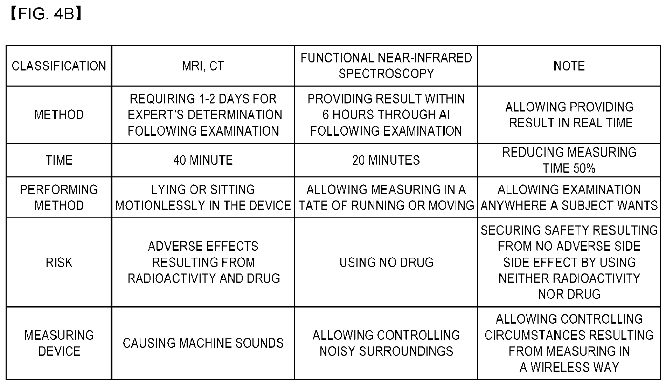

FIG. 4A and FIG. 4B are views for explaining fNIRS (functional near-infrared spectroscopy) to be adopted to the present invention.

Referring to FIG. 4A, in the fNIRS technique applied to the present invention, the fNIRS is an imaging technique which irradiates light of the near-infrared area having a wave length of 650 to 1000nm and detects light penetrating a brain tissue to measure a change of the hemoglobin concentration noninvasively according to a change in blood flow of the brain without any risk resulting from a surgery.

The fNIRS technique applied to the present invention uses a fNIRS for performing real-time brain measurement under live circumstances in which a patient is able to moving rather than being fixed.

The fNIRS is an imaging technique which irradiates light of the near-infrared area having a wave length of 650 to 1000nm and detects light penetrating the brain tissue to measure a change of the hemoglobin concentration non-invasively according to a change in blood flow of the brain without any risk resulting from a surgery.

This fNIRS may shows a result in real time differently from the existing assessment methods, reduce costs for buying a device and maintenance fee thereof, and increase reliability of a measurement result as the measurement result shows a sociality directly.

FIG. 4B shows a comparison between the current brain measuring technique and the fNIRS adopted to the present invention.

In the present invention, a non-identified MRI imaging scan of the brain is collected, an activated MRI imaging scan data of the brain and reviewed clinical data is collected, a feedback is received form a radiologist followed by organizing the same, and the clinical information is structuralized into a labeled imaging scan of the brain, a sheet file in excel format and the like through imaging preprocessing.

That is, collected are an inactivated MRI imaging scan of the brain for MRI diagnosis, clinical information and the like from the medical database operated by a hospital, this allowing defining accurate references for hearing loss, tinnitus and furthermore dementia by using imaging scan reading and pathological reading.

Further, a diagnosis result of the MRI imaging scan is classified into PSAP result data of hearing loss, tinnitus and normalcy and MRI imaging scan of the brain. In a case of hearing loss, tinnitus or normalcy, there may be a misdiagnosis at the time of reading. Thus, database may be constructed under a specific environment.

As a particular reference for hearing loss described hereinafter, there may be used an imaging scan of the brain of a patient diagnosed as hearing loss by a PASAP result. As a particular reference for tinnitus, there may be used an imaging scan of the brain of a patient diagnosed as tinnitus by a PASAP result. As a particular reference of normalcy, there may be an image scan of the brain of a patient diagnosed as normalcy by a PSAP result.

Further, a background service is registered, a questionnaire is filled out before visiting an institution, an interview on mood and vitality is conducted prior to brain measurement, a task for the brain measurement is selected followed by performing the selected task, and determination on whether or not to collect sufficient data is made either to finish the brain measurement or restart the preceding step.

Further, the imaging data adopted to the present invention may be defined as data of an activated MRI imaging scan of the brain.

Further, non-identification of the imaging data may be processes into a non-identifying tag structure (RAW data form) by a non-identifying method.

Further, the metadata adopted to the present invention collects patient's metainformation about imaging information and is used for quality inspection through additional information in a case of patients with hearing loss and tinnitus to secure reliability.

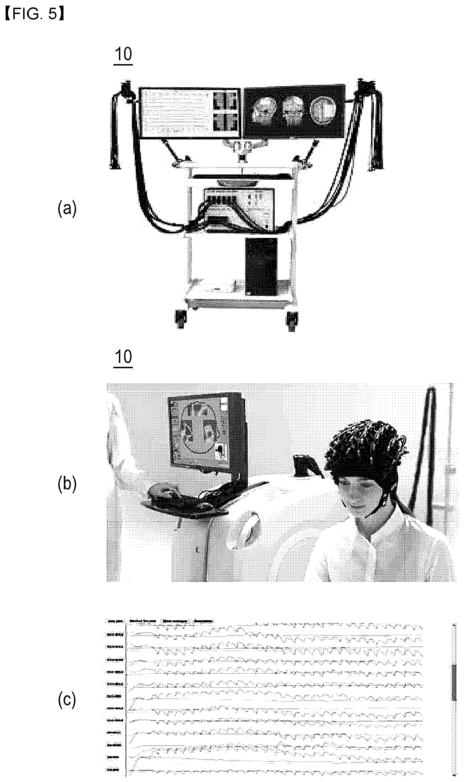

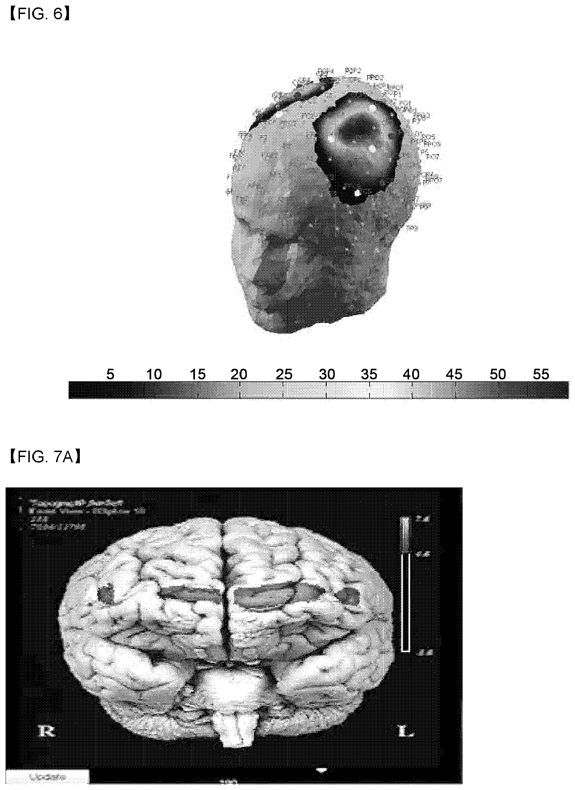

Meanwhile, FIG. 5 and FIG. 6 show one example in which signals measured by the brain signal measurement portion are visualized and displayed in connection with the present invention.

Referring to FIG. 5 and FIG. 6, it may be found that the brain activation area is checked safely without any surgery by irradiating near infrared ray to the brain, followed by detecting light penetrating the cerebral cortex and processing the detected light to measure concentrations of Oxy Hb and Deoxy Hb.

Further, the exemplary device neither has riskiness nor is affected by ambient noises. Further, this device has high convenience and portability and is cost-effective compared to other brain function imaging devices.

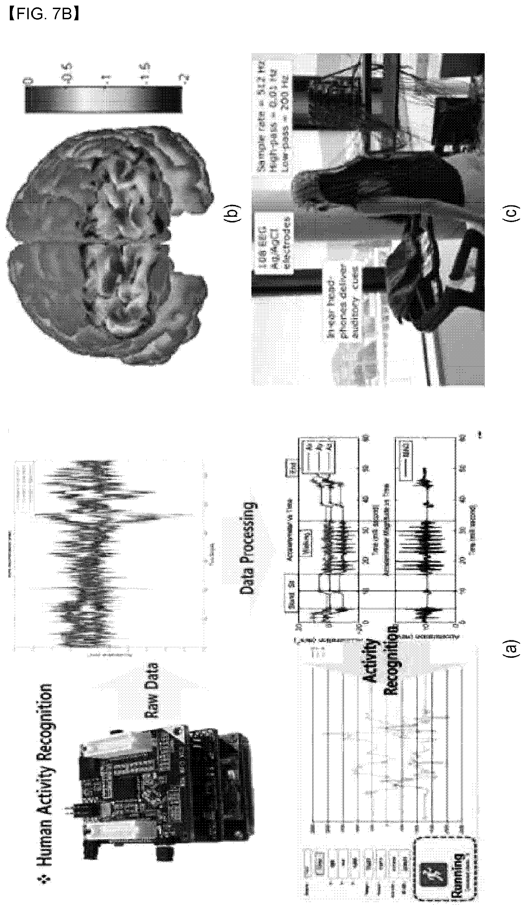

FIG. 7A to FIG. 7C show one example of each of the signals measured by the brain signal measurement portion, visualized information and processed information in connection with the present invention.

Referring to FIG. 7A to FIG. 7C, shown is a block average of time series of channel position dependent fNIRS signals.

Further, shown is a mapping of the block average of the channel position dependent fNIRS signals on the head.

In the present invention, in order for the application to machine learning, a data configuration for preventing algorithm bias and a method for collecting data may be used as follow.

Sample bias may occur when the collected data either represents an environment in which the AI system is expected to be performed or does not show this clearly.

Generally, algorithm learns a data set of a subset deliberately selected from a whole data set rather than learning the whole of the data set. Thus, in order to reduce the sample bias, it is important to select a subset that is capable of representing the whole of the data set besides sufficiently large one when selecting the data set of the subset. Further, measurement bias occurs when there is distortion problem in values by a device used in observing or measuring something, this kind of bias likely to distort the data in a certain direction.

An instrument with measurement bias fails to duplicate an environment in which a model will be operated. The learning data distorts actual data, this resulting in a biased result. The measurement bias is not avoidable even if collecting data more. Accordingly, in order to solve the aforementioned problem, the present invention configures an environment not allowing resulting in biased results to collect the data.

As a noninvasive method for measuring brain functions, the fNIRS measures a change in blood flow of the brain by using near infrared ray, the measurement principal thereof being based on measurement of a level of oxygenation of hemoglobin in the blood flow of the brain using the far infrared ray.

As irradiating near infrared ray to a living body and detecting and processing a fNIRS signal penetrating the tissue, it is allowable to measure an Oxy Hb concentration and a Deoxy Hb concentration.

The brain activation area consumes oxygen transferred by Oxy Hb in the blood flow and the Oxy Hb is changed into Deoxy Hb. Two such hemoglobins have an optical property that changes in a visible-ray area and a near-infrared area, and concentrations thereof measured by fNIRS are usable as a criterion for brain activity.

Such a method according to the present invention is capable of measuring brain functions in a state that a person is running or moving and of providing a result within a short period of time after examination.

Further, the method allows measuring the brain functions without any medicine and controlling ambient sounds.

In the present invention, in order to build an appropriate model, variables having the highest explanation ability is selected from variables which are determined to have a high correlation with a target item subjected to abnormality detection.

Further, adjust R^2 for each subset is measured to select an item made up of the least number of variables from the subset which has the largest value.

Further, an authoring tool is required in order to build AI learning data effectively.

That is, a labeling function is required which automatically tags and labels a target image subjected to labeling, a labeling list, a reference image and the like with labeling project information stored in the server with an imaging labeling application solution. As a way of the authoring tool for the operation management of the AI learning data, functions of adding, storing and generating an image may be used.

An image desired to be examined may be added using a relevant authoring tool. Following adding an MRI imaging scan of the brain or a fracture part by using a portable MRI device, functions of adding and generating an additional image may be grafted as pressing a plus button.

Further, an authorizing tool using an annotation function may perform a tagging operation for the additionally stored and generated image and an additional information recording operation through a note function for i.e. peculiarity and the like.

As a way of utilization for the operation management of the AI learning data, a cross validation may be performed to verify an exemplary reading model of normalcy, hearing loss and tinnitus for the activated MRI imaging scan of the brain.

Further, a performance is assessed by using various reading criteria, the criteria including measurement of sensitivity, specificity and an Area Under a Receiver operating Characteristic Curve (AUROC). The exemplary reading model may be built through github based thereon.

As mentioned above, the brain signal measuring portion 10 collects information related to the user's brain and the brain signal collecting operation of the brain signal measuring portion 10 is performed in a state that the user is moving.

Meanwhile, the diagnosing portion 50 determines a brain state of the user based on the collected signal.

In particular, the diagnosing portion 50 may determine the brain state of the user based on at least one brain activation area which was extracted successively for a predetermined period of time.

Herein, the diagnosing portion 50 may diagnose a brain disease of the user, the brain disease including dementia, Parkinson's disease, cerebral stroke, Epilepsy, brain cancer and developmental disabilities.

Furthermore, the management portion 60 may be used additionally which provides information for ameliorating the brain state of the user, corresponding to the brain state of the user' determined by the diagnosing portion 50.

For example, when the user, by applying preset references, is suspected of at least one of dementia, Parkinson's disease, cerebral stroke, Epilepsy, brain cancer and developmental disabilities within a certain period of time based on the brain state of the user, the management portion 60 controls the brain signal stimulating portion 20 to be described below to provide a signal for stimulating the user's brain for ameliorating the brain state of the user.

### Brain signal stimulating portion

The brain signal stimulating portion 20 provides a function of stimulating the user's brain for the signal collecting operation of the brain signal measuring portion 10.

Furthermore, the brain signal stimulating portion 20 may provide a function of stimulating the user's brain according to controlling of the management portion 10 for ameliorating the brain state of the user.

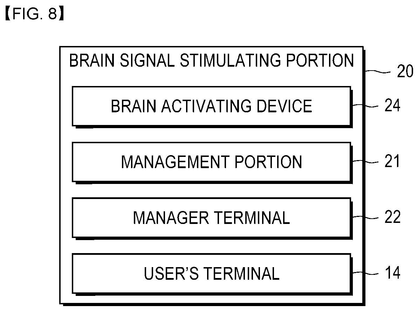

FIG. 8 is a block view of a brain signal stimulating portion that is a configuration element of the AI based device for providing brain information.

The brain signal stimulating portion 20 intensively stimulates a synapse meaning a part in which brain cells are connected to communicate with each other. In order to activate a brain area as long-term memory, the brain area should be stimulated through a place, emotion and a story.

Accordingly, the brain signal stimulating portion 20 reinforces an activity of a target brain area intensively through multidimensional environmental stimulations based on a natural object disposed in a space where activities, such as exhibition, music, motion, etc., are made and selectively activates a function of the brain area. According to classification criteria of Diagnostic and Statistical manual of mental Disorders (DSM-V) defined by American Psychiatric Association, the brain area may be classified into attention, visuospatial ability, memory, executive ability, verbal ability, calculation ability and sound cognitive ability, and the like. That is, the present invention intensively reinforces various activities of the brain area, such as attention, empathy, creativity, memory, healing, and the like through the natural object, particularly a plant and multi-dimensional environmental stimulation such as sound, sense of touch, scent, sense of sight, memory, and the like and activates functions.

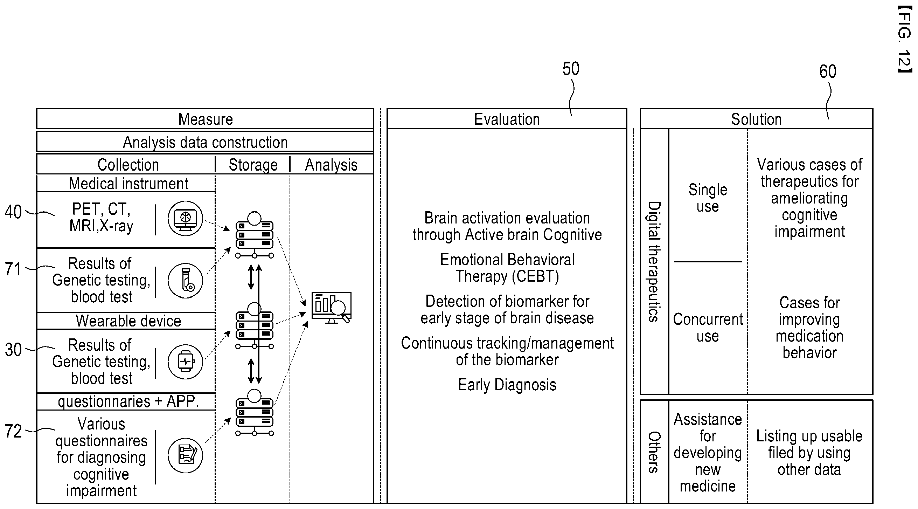

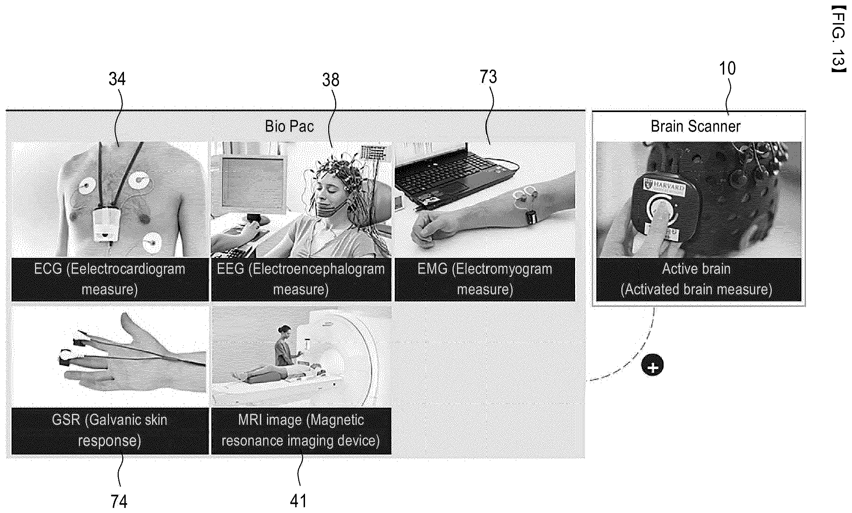

As shown in FIG. 12 and FIG. 13, a brain activation system 10 according to a preferable embodiment of the present invention includes a brain activating device 24 and a management server 21, the brain activating device 24 applying a multi-dimensional environmental stimulation systemized to intensively reinforce an activity of a pre-targeted brain area within a predetermined space where the natural object was installed and the management server 21 controlling driving of the brain activating device 24 to apply the multi-dimensional environmental stimulation to the targeted brain area in the space.

The brain activating device 24 may include a media portion 30, an acoustic portion 40, a lighting portion 50 and a scent transferring portion 60 in order to intensively reinforce the activity of the pre-targeted brain area within the predetermined space where the natural object was installed and to activate the function of the brain area, the media portion 30 implementing media art corresponding to a concept desired to be generated in the space, the acoustic portion 40 outputting a sound, the lighting portion 50 irradiating light and the scent transferring portion 60 transferring a scent to the user.

### User data collecting portion, Imaging obtaining portion. Diagnosing portion and Management portion

The user data collecting portion 30 collects body information related to the user.

The body information related to the user may include the user's auditory information, gait information, stress information, electrocardiogram information, sleep information, attention information, emotional change information, brain wave information, oxygen saturation information and the like.

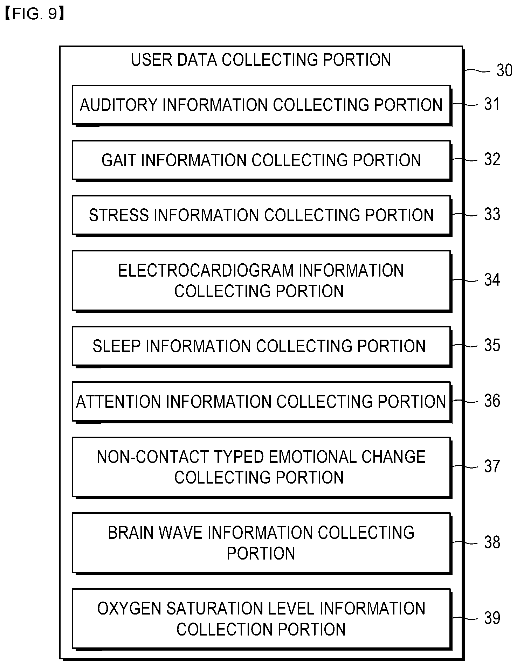

FIG. 9 is a block diagram of a user data collecting portion in connection with the present invention.

Referring to FIG. 9, the user data collecting portion 30 may include an auditory information collecting portion 31 that collects the user's auditory information.

Wherein, the diagnosing portion 50 determines a level of hearing sensitivity damage of the user based on the collected auditory information and may determine the brain state of the user by using at least one brain activation area together with the determined level of hearing sensitivity damage of the user.

Particularly, when the user's hearing sensitivity damage results from hearing loss, the diagnosing portion 50 may determine the brain state of the user by additionally using the body information related to the user, an average value of pure-tone thresholds in both of the user's ears, a level of the user's hearing loss and information related to the auditory information collecting portion.

As another example, when the user's hearing sensitivity damage results from tinnitus, the diagnosing portion 50 may determine the brain state of the by additionally using the body information related to the user, an average value of pure-tone thresholds in both of the user's ears, a level of the user's tinnitus and information related to the auditory information collecting portion.

Meanwhile, the brain signal collecting operation of the brain signal measuring portion 10 may be performed in a state that the user's hearing sensitivity damage occurs while the user is moving.

Further, the user data collecting portion 30 may include a gait information collecting portion 32 that collects the user's gait information, a stress collecting portion 33 that collects the user's stress information, an electrocardiogram information collecting portion 34 that collects the user's electrocardiogram information, a sleep information collecting portion 35 that collects the user's sleep information, a attention information collecting portion 36 that collects the user's attention information, a non-contact typed emotional change collecting portion 37, a brain wave information collecting portion 38 that collects the user's EEG(electroencephalogram) information and an oxygen saturation information collecting portion 39 that collects the user's oxygen saturation information.

Wherein, the diagnosing portion 50 may determine a level of the user's hearing sensitivity damage based on the collected auditory information and determine at least one of the user's gait pattern, a level of stress, an electrocardiogram change, a sleep state, an attention change, an EGG change and an oxygen saturation change based on the collected information.

Further, the diagnosing portion 50 may determine the brain state of the user by using the at least one of the level of the user's hearing sensitivity damage, the user's gait pattern, a level of stress, an electrocardiogram change, a sleep state, an attention change, an EGG change and an oxygen saturation change together with the at least one brain activation area.

Further, the signal collecting operation of the brain signal measuring portion 10 may be performed under the circumstance being applied with at least one of states that the user's gait information is changing, the user's stress is changing, the user's electrocardiogram is changing, the user's sleep condition is changing and the user's attention is changing, while the user is moving.

Further, the user data collecting portion 30 may include the non-contact typed emotional change information collecting portion 37.

This non-contact typed emotional change information collecting portion 37 may collect the user's face landmark masking data based on the user's eye tracking to collect the user's emotional change information in a non-contact way.

Wherein, the diagnosing portion 50 may determine the brain state of the user by using the at least one brain activation area together with the user's emotional change information.

Further, the non-contact typed emotional change information collecting portion 37 may collect the user's emotional change information based on the user's speech change. Wherein, the diagnosing portion 50 may determine the brain state of the user by using the at least one brain activation area together with the user's emotional change information.

Particularly, the diagnosing portion 50 may determine the brain state of the user based on the at least one brain activation area extracted successively for a predetermined period of time.

Wherein, the diagnosing portion 50 may determine the brain disease of the user, the brain disease including dementia, Parkinson's disease, cerebral stroke, Epilepsy, brain cancer and developmental disabilities.

Furthermore, additionally used is the management portion 60 that provides information for ameliorating the brain state of the user, corresponding to the brain state of the user determined by the diagnosing portion 50.

Hereinafter, described is the function of the diagnosing portion 50 using, as a representative of the user data collecting portion 30, the auditory information collecting portion 31 and the non-contact typed emotional change collecting portion 37.

Firstly, an embodiment using the auditory information collecting portion 31 is described in detail.

As the aged society results in a spectacular increase in the population with dementia and Korea enters the aged society, dementia becomes an issue in the youth population besides the aged population.

Particularly, the proportion of patients with severe dementia to overall dementia patients shows a reduction trend, i.e. 32% as of the year 2016 and 30% as of the year 2018, through medication treatment and various programs for severe dementia patients. This means that symptoms of the severe dementia patients are ameliorated whereas early stage of dementia shows a growing trend.

Noise is an unwanted sound considered unpleasant and loud to hearing. Sensorineural hearing loss caused by this noise is referred to as 'noise-induce hearing loss".

The root cause lies on damage of the sensory organ, that is, cochlea. Particularly, this often occurs as a consequence of damaged outer hair cells. A sound of 75dB or lower hardly incudes hearing loss, however, a sound pressure of environment noise in the office or conversation circumstance approximates 60dB, a noise level inside the cabin of buses and subway and restaurants approximating 80dB, the maximum volume of the earphones of MP3s or portable CD players approximating 100dB, a noise level of airplanes approximating 140dB and a noise level of gunfire approximating 170dB. When listening through earphones at a volume to the extent that the next person can hear, the volume level ranges approximately from 100dB to 115dB. Prolonged exposure to a noise of 85dB or higher may cause damages on ears.

Exposures to a sound pressure of 100dB for 15 minutes or longer without any protection equipment and to a sound pressure of 110dB regularly for 1 minute or longer cause risk of hearing loss. In the light of that ordinary noise level inside the cabin of buses and subway is 80dB, the youth should maintain music sound to 90db or higher in order to hear music, the hearing loss occurring as a consequence of repeated exposures thereto.

Sound intensity is determined by the amplitude of a sound wave and the scale for measuring intensity is decibel (dB) scale. For every 10dB increase in the sound intensity, a noise level is increased two times. Usually, a sound intensity of 75dB or lower does not affect hearing sensitivity damage.

As symptoms of noise-induced hearing loss, when a person has noise-induced hearing loss, he/she is likely to turn on a television, a radio and the like, at a high volume and is often referred to as 'Saojeong' in Korean meaning a person who is slow in understanding what someone says or is partially deaf.

Further, the person with noise-induced hearing loss hardly understands what the other person says accurately even in a little noisy surrounding. Commonly, he/she shows decrease in hearing at 4kHz and is more likely to be accompanied by tinnitus. Further, in addition to hearing sensitivity damage and tinnitus, the person with noise-induced hearing loss may feel unpleasant and anxiety, suffers from insomnia, headache and is stressed, this disrupting his/her normal life. Even worse, the noise-induced hearing loss affects pulsation and blood pressure to cause maldigestion and autonomic dysfunction.

The noise-induced hearing loss is diagnosed by performing pure-tone threshold test and an audiometry which measures hearing sensitivity accurately by a frequency, the audiometry including tinnitus test, optoacoustic emission test, loudness recruitment test, auditory brainstem response test and the like. When a loss of hearing sensitivity proceeding gradually following prolonged exposure to noises, particularly the loss to the sound of a high frequency of 4kHz appearing clearly, the hearing loss is diagnosed. The damaged hearing sensitivity due to the noise induced-hearing loss is basically irreducible because damaged auditory cells are not repaired. Particularly, if a difficulty in hearing occurs suddenly within a few days, this may be sudden sensorineural hearing loss and thus required are precise examinations as well as treatments with steroid hormones, vasodilators, antiviral agents and the like.

In such a case, it is necessarily required to relieve the ear for a certain period of time for the recovery thereof.

The sudden hearing loss is referred to as sensorineural hearing loss that occurs suddenly without any clear causes, this occurring in one ear commonly but also appearing in both ears extraordinarily. Occasionally, this is accompanied by tinnitus and dizziness.

Generally, the sudden hearing loss is regarded as an urgent disease and needs to start to admission treatment. Supposed causes of the onset may include viral infection occurring in the auditory nerve, blood flow disorder in the inner ear, disruption or damage to the cochlea, immune disorders of the inner ear, neuropathological disease, tumor, ototoxic drugs and the like.

Acoustic tumor may occupy 1 to 2% of patients with the sudden hearing loss. This is diagnosed through MRI and a curative rate thereof is high in a patient who was subjected to the treatment at an early stage through combined modality therapy of adrenal cortical steroids, vasodilators, blood flow improving agents, metabolism improving agents, sedatives and the like, as well as dietary therapy such as low salt diet and high protein diet, while taking a relief in a hospital.

According to the treatment result, generally, one third of patients out of total patient subjected to the treatment were completely recovered, while the symptom was ameliorated in one third of them and there was not treatment effect in the rest.

In a person with severe hearing sensitivity damage, a risk of dementia is five times higher than those who have no hearing sensitivity damage. According to Lancet report, the hearing loss occupies 3% of causes of the onset of dementia, ranked first among 9 potential risk factors thereof.

The hearing loss may be an early symptom of Alzheimer disease showing decline verbal ability in a state that there are background noises.

Further, the hearing loss increases a demand for cognitive resources and the brain has to work harder when auditory ability deteriorated.

Understanding and tries to understand conversations demand that the brain 'borrows' cognitive ability from other parts thereof, particularly memory. Generally, the information transferred to the memory decreases as receiving more information.

The hearing loss causes brain tissue remodeling and/or relative deprivation resulting in decline cognitive ability and the decline auditory ability often results in impaired social interaction and social engagement disorder.

Accordingly, there was a study result that the progression of dementia might be slowed 75% in a fashion of approaching the environment by decreasing a cognitive ability required for interaction and preventing hearing loss.

Further, it is prominent to detect and treat hearing loss early for preventing and reducing decline cognitive ability.

Risks of dementia in each of the elderly with mild hearing loss and severe hearing loss appear two times and five times higher, respectively as compared to the population with normal hearing ability (Lin, Albert, 2014).

30% of the population with hearing loss experienced tinnitus, additionally requiring the treatment of hearing loss together with the examination and treatment of tinnitus (Healthy hearing, 2019). As a result of examination over patients with tinnitus, the tinnitus affected an auditory function first, followed by affecting a cognitive function (particularly attention and thinking ability) and dementia, sequentially.

In conclusion, the tinnitus will likely lead to dementia (Mahoney, Rohrer, Goll, Fox, Rossor, Warren, 2010).

As a result of analysis of 18 literatures from 1996 to 2014, the tinnitus decreases cognitive intelligence while increasing anxiety and depression (Tegg-Quinn, Bennett, Elikelboom, & Baguley, 2016).

In conclusion, this leads to problems with hearing > sentence hearing > word hearing > overall comprehension > responses > mild cognitive impairment (MCI) in order.

When there is auditory damage at every 30dB, a predisposition to cognitive impairment approximates 2 times, this occurring when a problem arises in at least on of hearing, cognition and sight rather than sequentially (LEE, Su-jeong, LEE, Seung-jin, SONG, Ji-yeon, KIM, Hyeong-hee, 2014).

The user data collecting 30 may include the auditory information collecting portion 31.

Wherein, the diagnosing portion 50 may determine a level of the user's hearing sensitivity damage based on the collected auditory information and may determine the brain state of the user using the at least one brain activation area together with the determined level of user's hearing sensitivity damage.

Particularly, when the user's hearing sensitivity damage results from hearing loss, the diagnosing portion may determine the brain state of the user by additionally using the body information related to the user, an average value of pure-tone thresholds in both of the user's ears, a level of the user's hearing loss and the information related to the auditory information collecting portion.

As another example, when the user's hearing sensitivity damage results from Tinnitus, the diagnosing portion may determine the brain state of the user by additionally using the body information related to the user, an average value of pure-tone thresholds in both of the user's ears, a level of the user's hearing loss and the information related to the auditory information collecting portion.

Meanwhile, a signal collecting operation of the brain signal measuring portion 10 may be performed in a state that the user's hearing sensitivity is damaged, while the user is moving.

Moving on to the next, a function of the diagnosing portion 50 using the non-contact typed emotional change collecting portion 37 will be described.

Biodata for personal emotion recognition may be collected and the data argumentation may be made based on the non-contact typed emotional change collecting portion 37. Texts and facial expression data may be collected which show 6 types of basic feelings for various domains (age, gender, existence of accessories) in a uniform distribution. A basic emotion label may be tagged on the collected data and collect face landmark masking data including eye tracking.

Further, pose estimation imaging and images may be collected for understanding a target object's intension and a speech argumentation method may be provided for correcting the emotion data imbalance.

Regarding the biodata based personal emotion recognition, emotions may be recognized through a trained Deep Neural Network (DNN) model using the collected data.

In order to increase an emotion recognition performance, it is allowable to develop and apply preprocessing methods such as noise removal, speech part extraction and the like, and modules for selecting and extracting speech characteristics suitable for the emotion recognition is applicable.

Further, an utterance level emotion recognition model based on deep learning is applicable and extraction of time series of speech is allowable to infer a basic emotion for the target object data and to recognize personal emotion utilizing the speech data. Among deep learning methodologies, an emotion recognition AI model is applicable which utilizes recurrent neural network taking account of time series, a transformer using attention mechanism and the like. Optimal emotional recognition is allowable by using the designed emotion recognition AI model in machine learning and hyperparameter tuning method such as Bayesian optimization.

Further, it is allowable to recognize contours of eyes, a nose, lips, eyebrows and face, and also to extract an attention level from the collected eye tracking data and face landmark time series (it is allowable to use attention based artificial neural network due to using of consecutive data).

It is allowable to apply a social emotion recognition method through tracking personal emotion and daily situation and to represent emotion embedding advancement and emotions in consecutive spaces rather than classifying them. Also, it is allowable to apply a multi-modal emotion analysis and the like by combining emotion embedding extracted from sight, speech and natural language to an embedding space reflecting continuity of the time series data.

For the social emotion recognition, personal emotion extraction and vectorization methods are applicable by using speech based texts, d-vector based speaker embedding, prior-developed speech emotion recognition methods.

It is allowable to extract and apply characteristics applying Cepstral mean and variance normalization (CMVN) for suppressing performance decline for the speech of daily conversation with uneven tones resulting from emotions and also to apply a language model applying domain adoption capable of taking account of characteristics of the expression and word which are changeable according to situation information.

As the non-contact typed emotional change collecting portion 37, a non-contact typed emotional change collecting portion may be used which collects the user's face landmark masking data based on the user's eye tracking to collect the user's emotional change information in a non-contact way.

Wherein, the diagnosing portion 50 may determine the brain state of the user by using the at least one brain activation area together with the user's emotional change information.

Further, as the non-contact typed emotional change collecting portion 37 a non-contact typed emotional change collecting portion may be used which collects the user's emotional change information based on the user's speech change.

Wherein, the diagnosing portion 50 may determine the brain state of user by using the at least one brain activation area together with the user's emotional change information.

Particularly, the diagnosing portion 50 may determine the brain state of the user based on the at least one of brain activation areas extracted successively for a predetermined period of time.

Wherein, the diagnosing portion 50 may determine the user's brain disease, the brain disease including dementia, mild cognitive impairment (MCI), Parkinson's disease, depression, cerebral stroke, Epilepsy, brain cancer and developmental disabilities. Furthermore, a management portion 60 may be further used which provides information for ameliorating the user's brain state, corresponding to the user's brain state determined by the diagnosing portion 50.

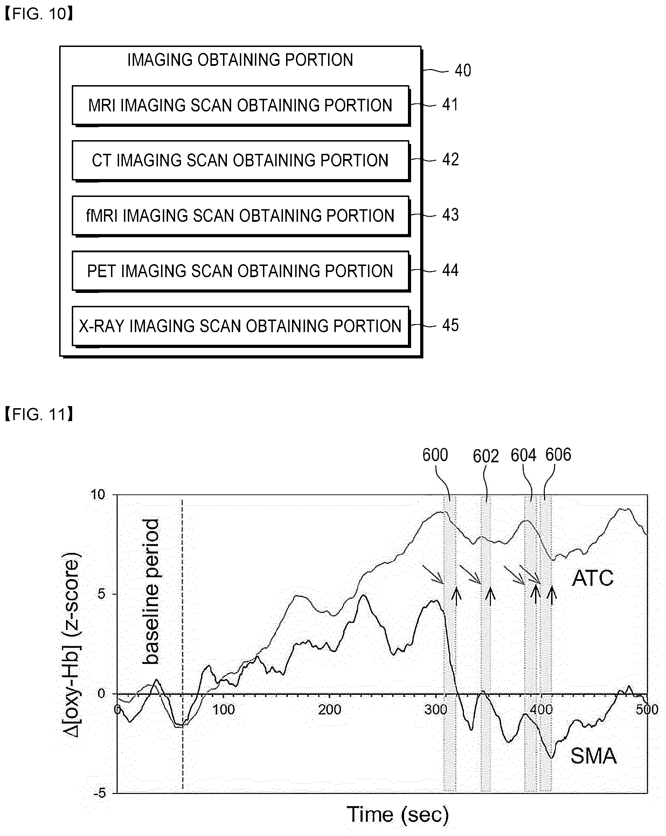

Meanwhile, FIG. 10 is a block diagram of an imaging obtaining portion in connection with the present invention.

The imaging obtaining portion 40 collects a medical imaging related to the user.

As the imaging obtaining portion 40, used are an MRI imaging scan obtaining portion 41 that collects an MRI imaging scan related to the user, a CT imaging scan obtaining portion 42 that collects a CT imaging scan related to the user and an fMRI imaging scan obtaining portion 43 that collects an fMRI imaging scan related to the user.

Wherein, the diagnosing portion 50 may determine the user's brain state based on the at least one brain activation area, body information related to the user and the collected medical imaging related to the user together.

The diagnosing portion 50 may determine the user's brain diseases including dementia, Parkinson's disease, cerebral stroke, Epilepsy, brain cancer and developmental disabilities.

Lastly, the management portion 60 provides information for ameliorating the user's brain state, corresponding to the user's brain state determined by the diagnosing portion 50.

For example, when the user is suspected of at least one of dementia, Parkinson's disease, cerebral stroke, Epilepsy, brain cancer and developmental disabilities by applying preset references based on the user's brain state, a user's brain stimulating operation of the brain signal stimulating portion 20 may be triggered for ameliorating the user's brain state according to controlling of the management portion 60.

### Diagnosing Portion based on the Collected Signals

Firstly, the brain signal stimulating portion 20 may generate a stimulating signal to stimulate the brain of a target object and output the generated stimulating signals. For example, the brain signal stimulating portion 20 may be a Head Mounted Display (HMD) device which provides Virtual Reality (VR) imaging but is not limited thereto. In various embodiments, the brain signal stimulating portion 20 may be one of various devices for providing a content for the brain stimulation in accordance with senses of sight, hearing, touch and the like.

When this brain signal stimulating portion 20 is an HMD device, the HMD device is mounted on the head of the target object and provides the target object a brain stimulating content for the virtual reality allowing spatial and temporal experiences similar to the reality. This may be also a complex VR experience device which detects physical, cognitive and emotional changes of the user in a state of experiencing the VR by obtaining the user's biodata. For example, the brain stimulating content may include non-interactive imaging such as movies, animations, advertisements, promotion imaging or the like and interactive imaging such as games, electronic manuals, electronic dictionaries, promotion imaging or the like but this is not limited thereto. Wherein, the imaging may be three-dimensional imaging and may include stereoscopic imaging.

This HMD device is formed into a structure capable of being mounted on the user's head and may be implemented into a form displaying various brain stimulating contents for the VR through a display portion inside the HMD device.

Further, when the HMD device has a display portion, one side of the display portion may be disposed facing the face of the target object, allowing the target object to confirm the brain stimulating content when wearing the HMD device.

The brain signal stimulating portion 20 according to various embodiments may further include a special equipment such as gloves, clothing and the like allowing the target object to feel sensuous effects like seeing and touching something in real and consequently to be immersed in the VR environment.

Next, the brain signal measuring portion 10 is to obtain fNIRS data generated by the brain stimulation of the target object and this may include a plurality of light sources used for obtaining the fNIRS data and a detecting portion corresponding to each of the plurality of light sources.

Particularly, the brain signal measuring portion 10 detects the plurality of light sources and the near-infrared ray from each of the plurality of light sources, and may include a detecting portion disposed as a pair with the each light source.

In this brain signal measuring portion 10, the light sources and the detecting portions are disposed in a form of a matrix of n x m (n and m are natural numbers) and this may be implemented into a form of a headpiece for enveloping the whole scalp of the target object. However, the brain signal measuring portion 10 is not limited thereto and may be implemented into various forms for detecting the near-infrared ray from the scalp of the target object. A channel is formed in the area between the light source and the detecting portion disposed as described above, allowing detecting the near-infrared ray within the formed channel.

The brain signal measuring portion 10 emits lights to the scalp of the target object through the plurality of the light sources, detecting a near-infrared ray corresponding to an SMA (supplementary motor area) which controls movement of the target object in near-infrared rays released from the scalp by the emitted lights, through the detecting portion which makes a pair with the each light source, allowing obtaining fNIRS data of the SMA. The brain signal measuring portion 10 may transfer the obtained fNIRS data to the diagnosing portion 50.

The diagnosing portion 50 determines a state of the target object by using the fNIRS data and may include at least one of a tablet PC (Personal Computer), a notebook computer and/or a personal computer.

Particularly, the diagnosing portion 50 computes analysis data for the SMA by using the fNIRS data received from the brain signal measuring portion 10, determining the state of the target object based on the computed analysis data. Wherein, the SMA plans movement of the target object, implementing and controlling the planned movement. For example, this may be activated when an individual is doing upper or lower workout, eye exercise, hand exercise or the like.

In order to compute the analysis data, the diagnosing portion 50 may perform power spectrum analysis and z-score analysis. However, this is not limited thereto and various data analysis methods may be used for determining the state of the target object. The diagnosing portion 50 may use at least one of a pattern mining method and a machine learning method to determine the state of the target object based on the analysis data computed through such a data analysis method.

When using the pattern mining method, the diagnosing portion 50 may generate an analysis pattern of the target object by using a pattern generating model trained for generating the analysis pattern based on the analysis data. Wherein, the pattern generating model may be a model based on a clustering algorithm.

The diagnosing portion 50 may determine the state of the target object by using a state predicting model trained to predict the state of the user stochastically based on the generated analysis pattern as described above. Wherein, when the target object is a patient with a specific disease and a stimulating content is provided for the treatment thereof, the state of the target object may mean a behavioral pattern that is changeable according to the provided stimulating content. For example, the diagnosing portion 50 may determine the state of the target object relating to exercise ability, sense of balance, learning efficacy, kinesthetic sense, movement planning and/or synesthesia, and the like, but this is not limited thereto.

When using the machine learning method, the diagnosing portion 50 may determine the state of the target object by using a predicting model for predicting the state of the target object based on the analysis data. For example, the predicting model may be based on models of DNN (Deep Neural Network), CNN (Convolutional Neural Network), DCNN (Deep Convolution Neural Network), RNN (Recurrent Neural Network), RBM (Restricted Boltzmann Machine), DBN (Deep Belief Network), SSD (Single Shot Detector) or a U-net based predicting model, but this is not limited thereto. The diagnosing portion 50 according to various embodiments may monitor the determined state as described above or provide feedback data for the determined state. For example, the diagnosing portion 50 may provide monitoring data or feedback data relating to exercise ability, sense of balance, learning efficacy, kinesthetic sense, movement planning and/or synesthesia, and the like.

A whole system according to various embodiments may diagnose the state of the brain such as brain activation, brain diseases and the like by measuring fNIRS data of the brain area to external stimulation in a state that the target object is moving.

As described above, the present invention determines the state of the target object by using the fNIRS data of the SMA that controls the individual's movement and provides the determined state as the monitoring or feedback data and thus allowing providing an appropriate stimulating signal to the target object with movement impairment such as a patient with Parkinson's disease or partial body paralysis caused by an accident for the treatment thereof and checking whether the brain of the target object works properly or not or the normality thereof spatio-temporally and in real time.

The brain stimulating portion 20 may include an imaging playing device and an input device. The imaging playing device is capable of wire/wireless communication with the input device. The brain signal stimulating portion 20 according to the provided embodiment means the brain signal stimulating portion (20) of FIG. 1 and will be assumed and described as the HMD device which plays VR imaging.

Firstly, the imaging playing device may include a communication part, a sensing part, a display portion, a storing portion and a control portion.

The communication portion allows the imaging playing portion to communicate with an external device. The communication portion is connected to the diagnosing portion 50 by using wire/wireless communication allowing transmitting/receiving various kinds of data. Wherein, the various kinds of data may be VR contents and these VR contents may be first- or third-person contents. For example, the third-person content may include an avatar matching the target object, the avatar implementing similar to the appearance of the target object.

The sensing portion includes a movement sensor for sensing the user head movement, allowing outputting a detecting signal by sensing the user head movement through the movement sensor. However, this is not limited thereto and various sensor for sensing the head movement may be used.

The display portion may show the user various contents (e.g. texts, images, videos, icons, banners or symbols). Particularly, the display portion may display the VR content received through the communication portion or stored in the storing portion.

The storing portion may store various kinds of data used for stimulating the brain. For example, the storing portion may store the VR content.

The storing portion according to various embodiment may include at least one type storage medium selected from a flash memory type, a hard disk type, a multimedia card micro type, a card type memory (e.g. an SD or XD memory and the like), a Random Access Memory (RAM), an SRAM (Static Random Access Memory, a Read-Only Memory (ROM), an EEPROM (Electrically Erasable Programmable Read-Only Memory), a PROM (Programmable Read-Only Memory), a magnetic random memory (MRAM), a magnetic disc, and an optical disc. The imaging playing device may be operated in relation with a Web storage that performs a storing function of the storing portion on the internet.

The control portion is connected operably with the communication portion, the sensing portion, the display portion and the storing portion, allowing executing various instruction for providing VR contents used for stimulating the brain of the target object. Particularly, the control portion may display the VR content received through the communication portion or stored in the storing portion through the display portion. For example, when the VR content is a third-person game content, the third-person game content may include an avatar corresponding to the target object. In this case, the control portion may provide a user interface for generating the avatar.

The control portion according to various embodiments may control the user interface related to the VR content by the operation of the input device by the target object. For example, the control portion controls the user interface for generating the avatar by the operation of the input device by the target object, allowing generating the avatar having an appearance similar to the target object.

The control portion according to various embodiments senses the movement of the head of the user through the sensing portion and plays the VR content corresponding to a direction of the head of the user, allowing displaying the VR content.

The input device (e.g. a controller and the like) is connected with the imaging playing device and the target object operates the input device connected to the imaging playing device, allowing controlling the user interface related to the VIR content displayed through the display portion of the imaging playing device.

The brain signal measuring portion 10 may include a light source, a detecting portion and a data transmitting/receiving portion.

This plurality of light sources is disposed in a form of a matrix of n x m and may emit light to the scalp.

The detecting portion may be provided as a pair with the corresponding light source and disposed in a form of a matric of n x m.

A channel is formed between the light source and the detecting portion disposed as described above, the detecting portion detecting near infrared ray in the channel to obtain the fNIRS data.

The data transmitting/receiving portion may transfer the obtained fNIRS data to the diagnosing portion 50 through the wire or wireless communication.

The diagnosing portion 50 may include a data receiving portion, a preprocessing portion, an analysis data computing portion and a state determining portion.

Firstly, the data receiving portion may receive the fNIRS data from the brain signal measuring portion 10.

The preprocessing portion may perform preprocessing for removing biological or technical artifacts. For example, the preprocessing portion may perform preprocessing by adopting a moving average low-pass filter. However, this is not limited thereto and various filters may be used for removing the artifact.

The analysis data computing portion collects preprocessed data corresponding to the channel related to the SMA at intervals of specific sampling time, allowing obtaining Δ data which shows a change in the concentration of oxy- and deoxy-hemoglobin based on the collected data.

The analysis data computing portion may generate analysis data by analyzing the Δ data. Particularly, the analysis data computing portion generates the analysis data by using two more analysis methods. For example, the analysis method may include the power spectrum method and the z-score analysis method.

Firstly, the analysis data computing portion may compute a power spectrum value by using the power spectrum analysis method to discern the area of the brain. For example, the analysis data computing portion converts time series data (i.e. the Δ data collected at intervals of specific sampling time) into a frequency domain using fast Fourier transform and computes power in the near infrared frequency window having a specific frequency range

Next, the analysis data computing portion for computing the analysis data using the z-score method may convert the fNIRS data, that is, x(t) ≡Δ into a time series z-score value (z(t)) by using an equation such as z(t) = [x(t)- M0]/SD0, wherein the MO and SDO may mean the average value of the Δ data and the standard deviation thereof, respectively for a reference time range(t).

The analysis data computing portion may compute the power value and the z-score value as the analysis data for the SMA by using the aforementioned analysis methods. The state determining portion may determine the state of the target object based on the computed analysis data. Particularly, the state determining portion may determine the state of the target object by using at least one of the pattern analysis method and the machine learning method. Herein, the state determining portion may determine exercise ability, sense of balance, learning efficacy, kinesthetic sense, movement planning and/or synesthesia and the like, as the state of the target object.

Firstly, when using the pattern analysis method, the state portion may generate an analysis data pattern of the SMA by using the pattern generating model trained to generate the analysis data pattern. Wherein, the pattern generating model may be a model based on the clustering algorithm. For example, in order to generate the analysis data pattern, the pattern generating model performs the clustering algorithm repeatedly to compute the average value of the silhouette for each of the plurality of SMA analysis data, allowing determining, as an optimum number of patterns, the number of patterns having the smallest value in the number of those having the most dramatically increased average values. Furthermore, the pattern generating model may generate one SMA analysis data pattern by grouping patterns for the plurality of SMA analysis data based on the determined number of patterns by using the clustering algorithm.

The state determining portion according to various embodiments may perform the clustering of a plurality of same SAM analysis data generated by computing the SMA analysis data for the target object at predetermined time intervals multiple times by using the pattern generating model.

The state determining portion according to various embodiments computes similarity and dissimilarity matrices for two sets of data selected from the plurality of SMA analysis data, allowing determining a correlation of the two sets of data based on the computed similarity and dissimilarity. Furthermore, the state determining portion 404 may generate one analysis data pattern based on the correlation of the analysis data and the optimum number of the patterns.

Next, the state determining portion may predict the state of the target object by using the state predicting model trained to predict the state of the target object stochastically based on the SMA analysis data pattern. Wherein the state predicting model may be a predicting model including steps of: computing SMA analysis data for a plurality of sample target objects different from the target object; generating patterns for each of the plurality of SMA analysis data by using the state predicting model; grouping each of the generated patterns to generate one analysis data pattern for each of the plurality of sample target objects; and determining a pattern related to the state of the target object in each of the generated analysis data.

The state determining portion may determine the state of the target object stochastically based on a comparison result obtained by comparing the pattern related to the predetermined state using the predicting model with the analysis data pattern for the target object. For example, the state determining portion 404 may determine exercise ability, sense of balance, learning efficacy, kinesthetic sense, movement planning and/or synesthesia, and the like.

Next, when using the machine learning method, the state determining portion may use a state predicting model pre-trained to predict the state of the target object based on the analysis data. For example, the state predicting model may be based on various learning models trained based on the analysis data of other target objects. For example, the predicting models used in various embodiments may be models of DNN, CNN, DCNN, RNN, RBM, DBN, SSD or the U-net based predicting model, but are not limited thereto.

In other words, the state determining portion may predict the state of the target object based on the input of the analysis data of the target object by using the state predicting model pre-trained to predict the state of the target object, that is, exercise ability, sense of balance, learning efficacy, kinesthetic sense, movement planning and/or synesthesia and the like.

The present invention as described above determines the state of the target object by using the SMA analysis data of the target object, allowing determining the target object as a patient with a brain disease or providing an index for preventing the brain disease. Hereinafter, a method for determining the state of the target object by using the fNIRS (functional near infrared ray spectroscopy) technique in an AI based device for providing brain information 1 will be described referring to FIG. 5.

The AI based device for providing brain information 1 obtains fNIRS data of the SMA which controls the movement of the target object by performing brain stimulation for the target object. Particularly, the AI based device for providing brain information 1 performs the brain stimulation to the target object by the brain signal stimulating portion 20 and may obtain fNIRS data for the SMA of the target object through the brain signal measuring portion 10. Herein, the fNIRS data for the SMA is obtained in an SMA related channel in the channels formed between the light source and the detecting portion making a plurality of pairs with each other and may be transferred to the diagnosing portion 50.

The AI based device for providing brain information 1 computes SMA analysis data by using the obtained fNIRS data (S510). Particularly, the AI based device for providing brain information 1 may calculate the SMA analysis data by using the power spectrum analysis and the z-score analysis. These calculating methods may be performed as described above.

The AI based device for providing brain information 1 determines the state of the target object based on the calculated analysis data. Particularly, the AI based device for providing brain information 1 may determine the state of the target object by using at least one of the pattern analysis method and the machine learning method through the diagnosing portion 50. The determined state as described above may be provided as feedback data for treating the disease of the target object or delaying progression thereof or monitoring data for preventing the disease. Further, the user may check whether the brain of the target object works properly or not or the normality thereof spatio-temporally and in real time by using these feedback data and monitoring data. Hereinafter, a method for determining the state of the target object based on the SMA analysis data calculated by using the z-score method will be described referring to FIG. 11.

FIG. 11 is an exemplary view showing analysis data in an SMA by using the z-score analysis method according to the embodiment of the present invention.

Referring to FIG. 11, the analysis data may be represented as a time series z-score value in the SMA. Generally, the SMA is an area that plans and controls the movement of the target object. Thus, when the target object performs a certain movement, the activity of the SMA is increased so that the time series z-score value of the SMA is gradually increased with reference to a reference line (z-score=0).

When a specific event for the target object occurs, the time series z-score value is decreased to be a smaller value than the reference line in a section 600, 602, 604, 606 where the specific event occurred. Herein, the specific event means that becomes acquainted with the movement of the target object. As the target object learns the movement quickly, a gradient of the time series z-score values may be declined sharply.

Even when there is a limit in the movement of the target object or the target object conducts a movement that is difficult to be performed, the SMA activity of the target object may be increased. For example, when the target object performs a new movement or exercise (e.g. tennis, swimming, boozing and the like), the SMA activity in charge of the movement is increased, allowing activating a plan to become acquainted with the new movement or exercise in the SMA. Thus, the target object may acquire or perform the new movement or exercise. As the target object becomes acquainted with such the movement or exercise, the activity of the SMA is gradually decreased, allowing performing this movement or exercise less actively, more naturally and with less planning and effort. Further, in a case of a patient with Parkinson's disease or partial body paralysis caused by an accident, the brain signal stimulating portion 20 is fitted on the head of the patient to play various brain stimulation contents through the brain signal stimulating portion 20, allowing providing a stimulating signal for treating the brain disease of the patient. In this case, the diagnosing portion 50 computes the analysis data for the SMA of the patient based on the fNIRS data obtained from the brain signal measuring portion 10, determining the state of the patient by using the computed analysis data and then providing the brain stimulating content for the treatment based on the determined state of the patient. In a case of the patient with Parkinson's disease or partial body paralysis, since the SMA has a feeble activity or is inactivated, the brain stimulating content is provided for gradually increasing the SMA activity of this patient through the brain signal stimulating portion 20, allowing helping treatment of the patient.

As described above, the state of the target object is determined by using the fNIRS data in the SMA related to behavioral implementation such as exercise ability or sense of balance, allowing diagnosing the target object as having the disease that decrease the SMA activation.

Further,the determined state of the target object is provided as the monitoring data or the feedback data, allowing generating an appropriate stimulating signal for preventing or treating the brain disease such as Parkinson's disease and the like.

Further, whether the brain of the target object works properly or not or the normality thereof may be checked spatio-temporally and in real time by using the analysis data in the SMA.

### Multisensor Biodata Based Model for providing activated XR Digital therapeutics

FIG. 12 is a view for explaining a multisensor biodata based model for providing activated XR digital therapeutics.

Herein, the XR environment may include one selected from AR (Augmented Reality), VR (Virtual Reality) and MR (Mixed Reality).

Referring to FIG. 12, the multisensor biodata based model for providing activated XR digital therapeutics is composed of three steps as shown in FIG. 12.

### Measure -> Evaluation -> Solution

In the measure step, it is allowable to collect medical instrument (PET, CT, MRI, X-Ray) information, results of genetic testing and blood test and the like.

Further, the information is collected through a wearable device (smart watch, band, ECG patch) and information additionally obtained through questionnaires and an application may be used.

Next, in the evaluation step, it is allowable to adopt the brain activation evaluation through Active Brain, brain activation maximization through the XR environment, Cognitive Emotional Behavioral Therapy (CEBT) and the like.

Further, it is allowable to use this information as a biomarker for early stage of brain disease or to adopt a function of continuous tracking/management of the biomarker. Furthermore, the information may be used for early diagnosis.

Lastly, in the solution step, is it allowable to use the aforementioned information as a plurality of digital therapeutics for the amelioration of cognitive impairment and the like and to improve medication behavior through a concurrent use thereof.

Further, it is allowable to use the information in new medicine development.

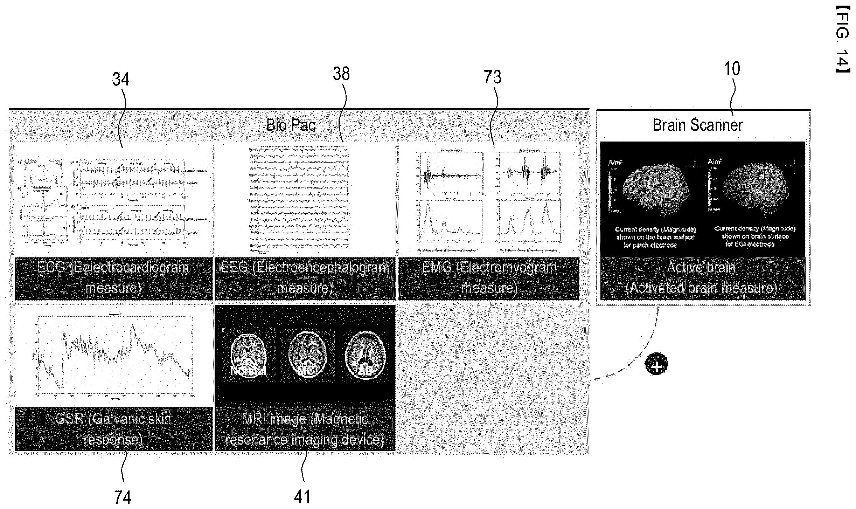

FIG. 13 and FIG. 14 show one example of each biodata obtained according to the present invention in association with the main device and data used for obtaining the biodata in the "Measure" step.

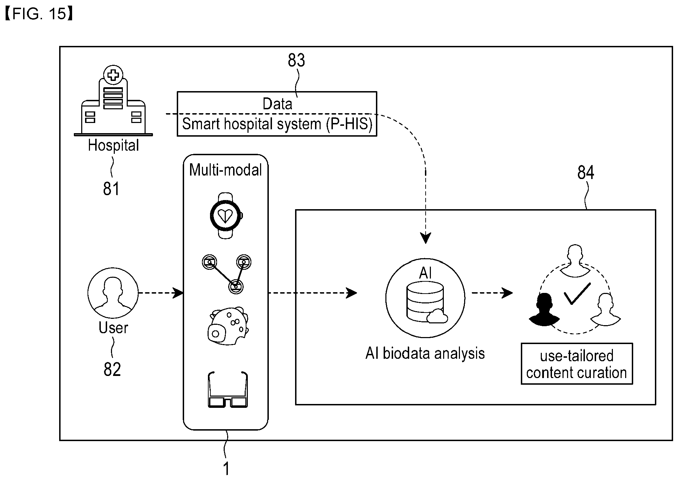

Meanwhile, FIG. 15 is a view for explaining one example which adopts the digital content-based method and system for providing therapeutics information according to the present invention.

In FIG. 15, shown is one example of a configuration of a multimodal biodata based XR content platform in connection with a smart hospital system.

Referring to FIG. 15, measured is a dynamic activation state of the brain when taking a rest as well as when conducting a certain behavior, collected and analyzed are a variety of brain related biodata and verified is the biodata for the brain activation in the XR environment.

Further, it is allowable to adopt a Cognitive Behavioral Therapy (CBT) method capable of defining a brain area that affects depression in the XR environment and of activating the relevant region and to proceed not only to develop the therapeutic XR content but also to experience with the XR content simultaneously with measuring the brain in a dynamic state and collecting the biodata suing various biosensors, allowing providing individually tailored therapeutics and inducing the increment of therapeutic effect. Henceforth, the model of measure + therapeutics will be new data that has not ever been used in the existing diagnosis of depression, that is, a therapeutic module may be used as a preventing module.

### Method for Providing Digital based Therapeutics

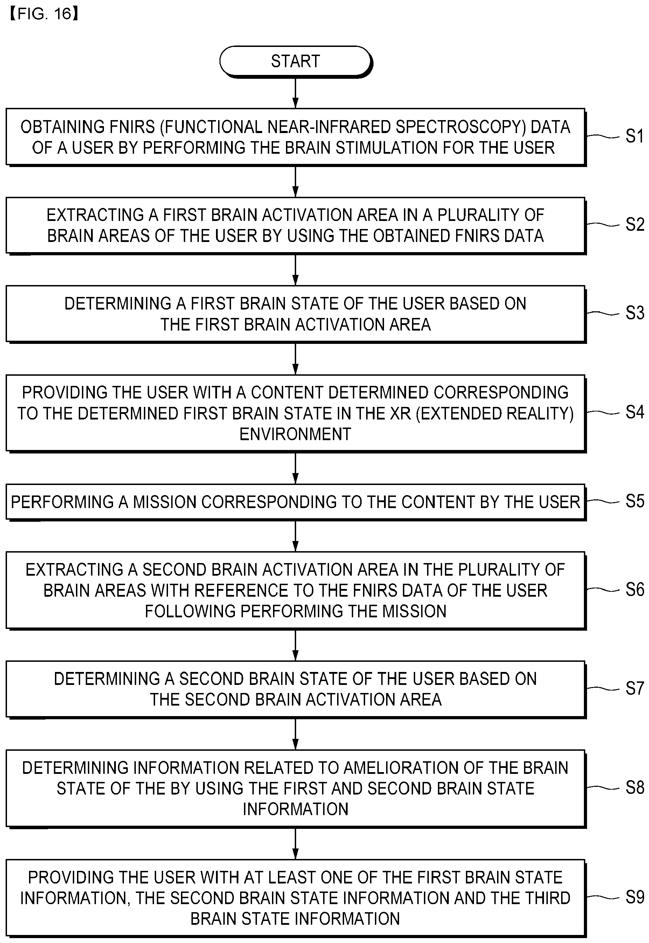

FIG. 16 shows one example of a flowchart for explaining the digital content-based method for providing therapeutics information.

Referring to FIG. 16, firstly, the brain stimulation for a user is performed to obtain fNIRS (functional near-infrared spectroscopy) data of the user S1.

Next, a first brain activation area is extracted in a plurality of brain areas of the user by using the obtained fNIRS data S2.

Following the S2, a first brain state of the user is determined based on the first brain activation area S3.

In the S3, a brain disease of the user is determined based on the first brain state of the user, the brain disease including mild cognitive impairment (MCI), Parkinson's disease, depression, cerebral stroke, Epilepsy, brain cancer and developmental disabilities.

Prior to the S1, the method may further include a step 0.5 of collecting body information related to the user and medical imaging information related to the user. In the step 3, the first brain state of the user may be determined by using information of the first brain activation area together with information the body information and the medical imaging information.

In the step 3, the first brain state of may be determined by additionally using a correlation of activation information related to each of the plurality of areas, the body information and the medical imaging information based on pre-accumulated database. Herein, the body information may include at least one of a degree of hearing loss of the user, gait pattern of the user, a stress level, an EGG change, a sleep state and attention change information, oxygen saturation change and the like.

Further, the medical imaging information may include at least one of an MRI imaging scan related to the user, a CT imaging related to the user and an fMRI imaging scan related to the user.

The S1 may include a S1-1 of emitting light to a scalp of the user through a plurality of light sources and a S1-2 of detecting the fNIRS data from the scalp to which the light was emitted through a detecting portion corresponding to each of the plurality of light sources.

Herein the fNIRS data represents a change of NIR light intensity transmitted through the scalp and may be data from which artifact was removed through a moving average low-pass filter.

Further, in the step 3, a power spectrum method and a z-score analysis method may be used for determining the first brain state.

Further, in the S3, analysis data for an SMA (supplementary Motor Area) which controls the user's movement based on the first brain activation area may be calculated to determine the first brain state based of the calculated analysis data.

Further, in the S3, in the situation that a specific event happened to the user, it may be determined that there is a limit in the movement of the target object as an analysis data value for the SMA is increased with reference to a reference line, whereas it may be determined that the target object is acquainted with the movement as the analysis data value is decreased with reference to a reference line.

Meanwhile, in the XR (extended Reality) environment, a content determined corresponding to the determined first brain state is provided to the user S4.

The S4 may include a S4-1 of determining the first brain state as a base line; a S4-2 of determining the content for activating the first brain activation area, corresponding to the first brain state and a S4-3 of providing the user with the content through a device which the user wears, allowing the user to experience the XR environment.

Further, the content may include a relaxation content for stabilizing emotion, a problem-solving content for controlling a stressed circumstance, an engagement content, a self-control content for controlling emotion and an impulse control content for controlling an impulsive behavior.

Further, the user may be plural in number and the content may further include a group therapy in which at least a part of the plurality of users performs a mission together.

Further, in the step 3, a brain disease of the user may be determined, the brain disease including dementia, MCI, Parkinson's disease, depression, cerebral stroke, Epilepsy, brain cancer and developmental disabilities.

Further, in the step 4, the content may be determined relating treatment of the determined brain disease.

Further, the user performs a mission corresponding to the content S5.

The S5 may include a S5-1 in which the user performs the mission changeable depending on a play processing level of the content and a S5-2 of providing information related to the brain of the user in real time while the user is in a state of performing the mission.

Further, even not shown, the S5 may further include a step of extracting a third brain activation area from the plurality of brain areas with reference to fNIRS data of the user while performing the mission and determining a third brain state of the user based on the third brain activation area.

Further, a second brain activation area is extracted in the plurality of brain areas with reference to the fNIRS data of the user following performing the mission S6.

Further, a second brain state of the user is determined based on the second brain activation area S7.

Further, information related to amelioration of the brain state of the user is determined by using the first and second brain state information S8.

When the step 8 further includes a step of extracting the activated third brain activation area in the plurality of areas with reference to the fNIRS data of the user in the middle of performing the mission and determining the third brain state of the user based on the third brain activation area, the step 8 may determine information related to the amelioration of the brain state of the user by using the first brain state information, the second brain state information and the third brain state information together.

Further, the user is provided with at least one of the first brain state information, the second brain state information and the third brain state information S9.

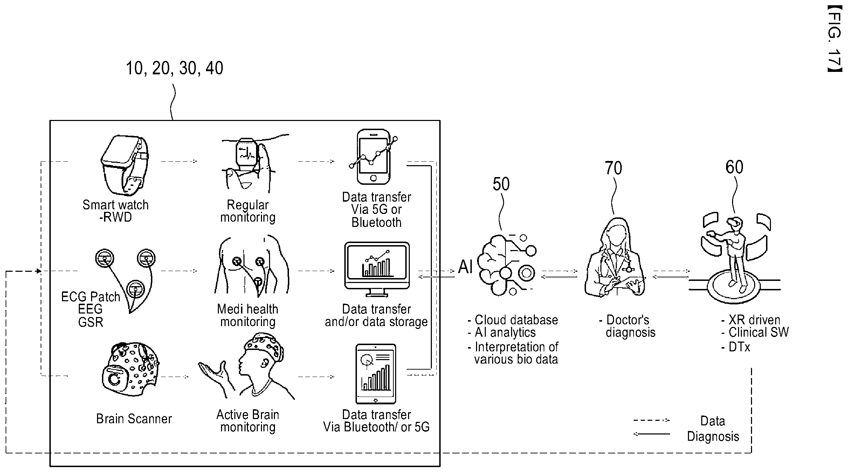

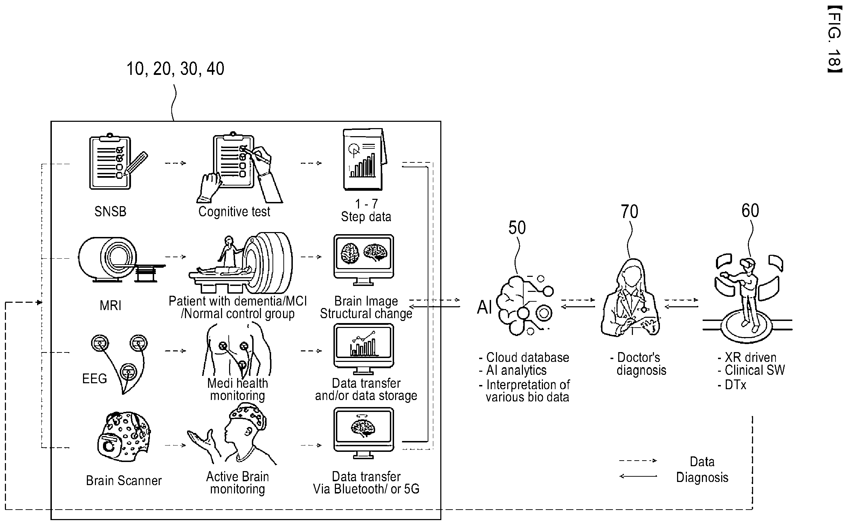

FIG. 17 and FIG. 18 show views for explaining a technique for predicting, diagnosing and treating a disease, based on processing of a correlation of a plurality of data in connection with the present invention.

Referring to FIG. 17, it is allowable to measure data of memory, judgement, attention, emotional changes and stress for a normal control group based on fNIRS, ECG, EEG, GSR and EMG, to verify a correlation of data of fNIRS, ECG, EEG, GSR and EMG and to perform a curing process through the digital therapeutics in the XR content applied environment. It is allowable to verify a correlation of Active Brain data (fNIRS) with other biodata (ECG, EEG, GSR and EMG).

In FIG. 17, following wearing an Active brain scanner, a Smart watch, an ECG Patch and the like, an action task is performed in the XR content applied environment, allowing obtaining the data of fNIRS, ECG, EEG, GSR and EMG of the normal control group.

Further, it is allowable to determine the memory, judgement, attention, emotional changes, and stress changes of the normal control group while performing the action task.

Further, it is allowable to verify the correlation of the Active Brain data (fNIRS) with other biodata (ECG, EEG, GSR and EMG).

Further, following wearing the Active brain scanner, the Smart watch, the ECG Patch and the like, a cure task is performed in the XR content applied environment, allowing verifying effects of memory increment, judgement improvement, attention increment and emotional change and stress decrement in the normal control group through brain activation and CNS efficacy increment while performing the cure task.

Further, it is allowable to verify the correlation of the Active Brain data (fNIRS) with other biodata (ECG, EEG, GSR and EMG) while performing the cure task.

Meanwhile, referring to FIG. 18, it is allowable to measure data of memory, judgement, attention, emotional changes and stress for each of a dementia group, an MCI group and a normal control group based on fNIRS, ECG, EEG and GSR, to obtain brain MRI imaging scan data and SNSB data and to verify a correlation of Active Brain data (fNIRS), other biodata (ECG, EEG, GSR), MRI imaging data and SNSB data according to the classification into the dementia group, the MCI group and the normal control group.

In FIG. 18, following wearing an Active brain scanner, a Smart watch, an ECG Patch and the like, an action task is performed for each of the dementia group, the MCI group and the normal control group in the XR content applied environment, and SNSB is performed, allowing obtaining the data of fNIRS, ECG, EEG, GSR, EMG, SNSB and brain MRI imaging scan of each of the dementia group, the MCI group and the normal control group.

Further, it is allowable to verify the data of fNIRS, ECG, EEG and GSR for each of the dementia group, the MCI group and the normal control group while performing the action task, and to verify a correlation of the data of fNIRS, ECG, EEG and GSR with the SNSB data for each of the dementia group, the MCI group and the normal control group.

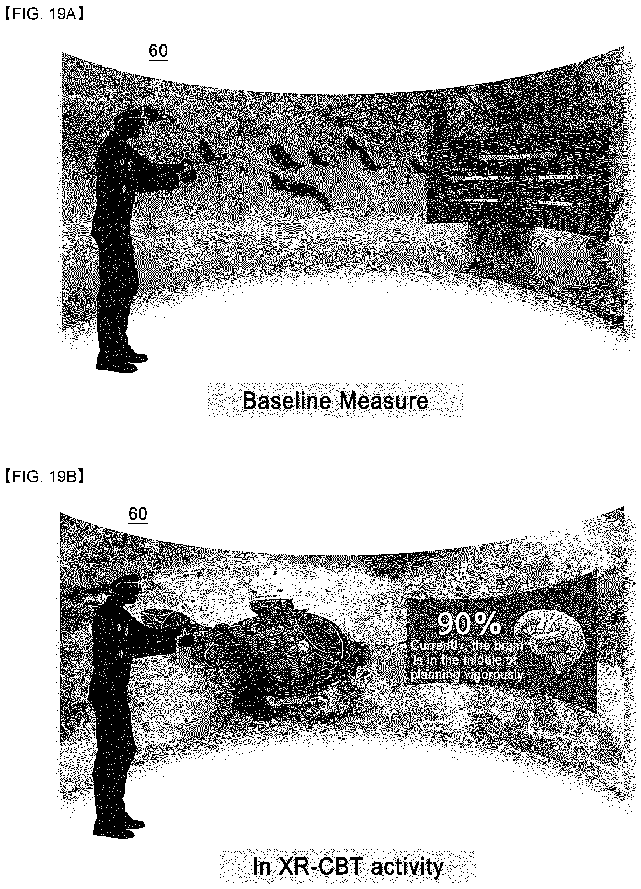

FIG. 19A to 19C show one example for providing a content determined corresponding to the brain state of the user under an XR (Extended Reality) environment.

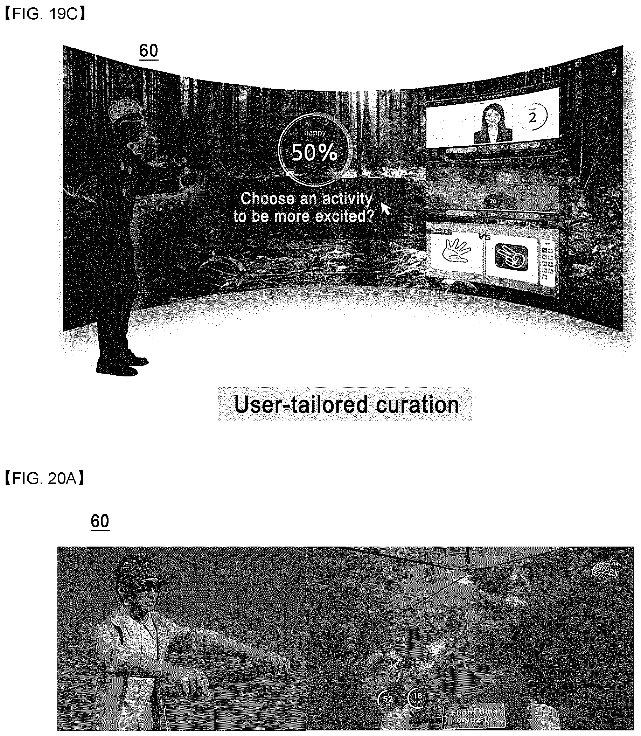

FIG. 19A shows one example of a baseline measure, FIG. B shows one example for processing an XR-CBT activity and FIG. 19C shows one example of a user-tailored curation.

That is, the user wears AR glasses for biosensor & XR experience such as Active brain scanner, a smart band, an ECG patch and the like. The brain state of the user of the baseline and the multimodal biodata is measured with the XR content having a gentle background, allowing diagnosing a level of depression and showing the user the depression level.

Further, the individually tailored content is configured based on the measured data.

Multiple kinds of XR activities and CBD methodologies are combined, 20 or more various XR-CBT content combinations are curated in order to most effectively activating the real time brain state of an individual and then the curated content is provided to the user.

Further, as processing the content and showing the state and activity of the brain and the biodata in real time in a state that the user takes movement, the user may confirm the effect on the brain him- or herself.

Further, following experiencing one content, a content to be proceeded next is curated and then the curated content is provided to the user. It is allowable to confirm the therapeutic effect by providing baseline measure data after completing the content experience and a result for comparing and analyzing the data following the experience. Accordingly, it is allowable to configure and provide a user tailored effective therapeutic content by collecting and analyzing brain activation data collected by the Active Brain scanner and a variety of biodata (ECG, REP and the like) collected by the multimodal biosensor for the brain state when using the XR content, on the basis of technical skills capable of measuring the brain activation state when the user is moving or takes a certain task.

Further, the XR content which the user experiences are combined with the CBT methodology, accompanying therapeutic effects and maximizing engagement and embodiment. This gives stimulation by using a gamification method, allowing activating the brain of the user at the most with a short period of time.

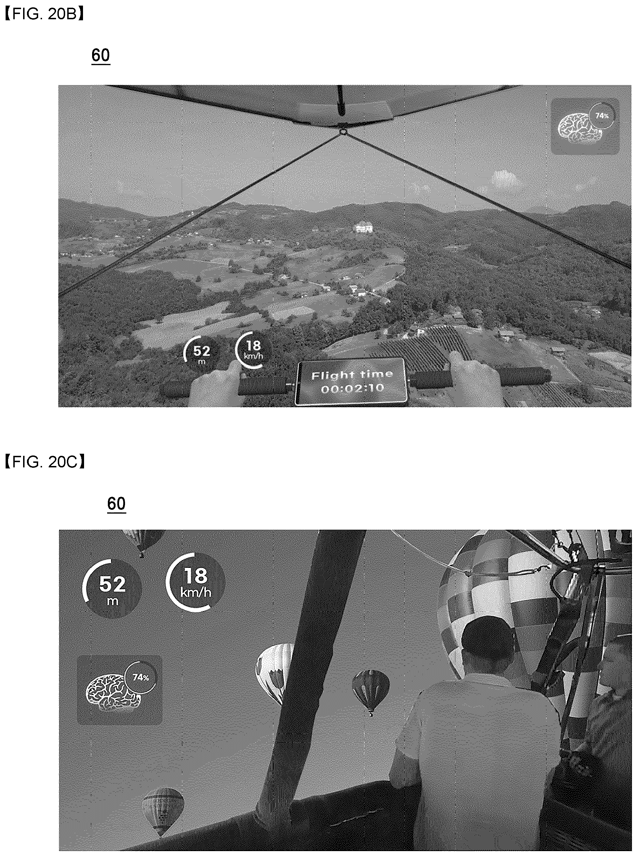

Further, as measuring an activated brain (by MRI and the like) rather than when taking a rest, it is allowable to contribute more precise treatment, that the user confirms a region of brain activation and a degree thereof him- or herself in real time, to provide and verify the user tailored XR-CBT content curation and to use this for early diagnosing and treating depression by combining measures of a variety of biodata. FIG. 20A to FIG. 20C show one example for that the user performs a mission corresponding to the content provided under the XR environment and monitors the brain state.

As shown in FIG. 20A to FIG. 20C, XR-CBT combined content may be used for the purpose of therapy.

The CBT is a psychotherapy method for treating maladaptive behavior by systemically integrating concept·principal·theory related to a cognitive aspect such as thinking·belief·value and an aspect of psychomotor behavior. A consultant uses this psychotherapy method in order to lead a consultee out into affirmative behavior and thinking changes using various skills.

In a case of depression, it is one way to hele a person with depression to recognize and change negative thinking patterns or behaviors. The depression CBT may be to concentrate on a currently facing problem and on how to solve this problem and to train up a new skill in the real world.

Further, it was revealed that Online CBT helped ameliorating depression symptoms similarly to an antipdressant through various study results.

The Online CBT has not been distributed broadly within the country while this Online CBT is actively used as a depression treatment/management method abroad.

Further, Gamified CBT is gamified with a computerized therapy method, allowing proceeding the CBT effectively with minimization of face-to-fact contact. As increasing the user's participation and maximizing engagement and amusement, it is allowable to provide a new user experience and creating therapeutic effects by inducing attention improvement, learning promotion and affirmative behavior changes.

The XR according to the present invention may improve recovery and healing effects by providing the user with an extended experience through engagement and coexistence in the XR.

As motivating the user in the VR environment, it is allowable that the user successfully completes repeating training possibly boring.

Further, as configuring the optimum environment capable of lowing psychological anxiety and stress, it is allowable that the user experiences highly concentrated meditation and psychological healing.

In case of the existing CBT, many sessions should be proceeded for a long period of time, engagement and self-participation are low and it is difficult to confirm self-state and therapeutic effects. Thus, there is a limit in motivating the user to undergo treatment.

It is very important to increase engagement in order for 'brain activation' for depression therapy. When proceeding the XR experience by providing content experience in a specific space in an appropriate environment, engagement affecting behavioral changes is maximized, allowing effective brain state measure as well as using this XR as a therapeutic means.

XR-CBR combined DTx according to the present invention takes advantages of engagement, coexistence and the like from the existing XR environment, having potential therapeutic effects similar to the online CBT and extending a therapeutic range through XR-CBT content curation according different symptoms and brain activation states of the user.

### Types of Contents

### (1) Relaxation

It is verified that relation behavior and training have a high effect on amelioration of symptoms of anxiety and depression by meta-analysis. The anxiety symptom is greatly decreased by maintaining composure and controlling emotion, this helps incapacitation of excessive worrying and negative thinking.

### (2) Problem-solving

Problem-solving therapy trains abilities for managing a stressful situation. Negative effects of the stress are lowered through solving a problematic situation and this increases self-efficacy.

### (3) Engagement

The user's participation is increased to improve attention and learning effect. As preparing a platform for learning techniques (abilities) in order for the user to feel like experiencing real-situation in the VR, it is allowable to increase engagement and entertainment more.

### (4) Self-control

Since the user's emotion and response is confirmed in real time through Active Brain and multimodal data, the user controls his or her emotion and behavior by checking effects or emotion resulting from his or her behavior (or inaction), consequentially reducing anxiety and worrying.

### (5) Group therapy

As the user has a feeling like performing a task with other peoples rather than doing the task alone, the user is encouraged in interaction with the others and has a feeling of gratitude that is one of affirmative psychological factors. As expressing one's gratitude, it is allowable to causes feeling of wellbeing and happiness and ameliorates symptoms related to depression and anxiety.

### (6) Impulse Control

Like a game raising blue and white flag, the user should repress an impulse until an appropriate moment according to an instruction is coming, allowing increasing his or her attention in a situation requiring concentration on the instruction and improving impulse control ability. As paying attention and changing an attentive object properly according to changes in the situation, it is allowable to improve an ability to convert attention.

### Content Scenario

(1) Prior to starting a content, a content may be configured for the user to confirm a self-state and a user tailored content is recommended based on the relevant contents.
(2) It is allowable to receive and experience the content that is suitable for one's situation. The content is configured to recommend next content in a connectional manner through the relevant contents by receiving data in real time during the experience, allowing maximizing therapeutic effects.
(3) Following completing the content, any change of the user is confirmed through comparing and analyzing the state with the data before experiencing the content, allowing applying this to the treatment through data set based analysis.

20 or more various kinds of different XR-CBT are configured by matching CBT methods applicable to each of various kinds of XR activities. Those contents are curated in a user tailored way based on multimodal biodata obtained by an Active Brain Scanner and biosensors, allowing inducing the user's continued participation in the treatment without boring in a circumstance requiring repetitive treatment or re-visit.

As one example, in a case of experiencing a rafting-relaxation content, it is allowable to confirm whether the user's emotion is stabilized or not and real time brain state of the user by Active brain screening and biodata. Also, the user may confirm the state his- or herself, allowing realizing the effect of the content.

After experiencing the content with the collected data, next content is curated. Since the user experienced the rafting activity already, a content is selected from contents of Baseball-Group Therapy or Fishing-Impulse Control, allowing informing the user of expected effects through the selected content and the user to experience a new activity and to confirm achievement.

Following completing all experiences, the user feels therapeutic effect by confirm a result of comparing and analyzing data for before and after those experiences.

### Effect According to the Present invention

When the aforementioned configurations are applied, the present invention is capable of providing a digital content-based device for providing therapeutics information.

Particularly, the present invention is capable of providing the device for providing digital therapeutics by collecting signals related to a brain of a user in a state of activity and determining a brain state of the user based thereon.

Further, the present invention is capable of providing the device for providing digital therapeutics by performing stimulation on the brain of the user to obtain fNIRS data, extracting an activation area in a plurality of brain areas using the obtained fNIRS data, determining a brain state of the user based on the brain activation area and providing a determined content under an XR (extended reality) environment, allowing the user to perform a mission corresponding to the content.

Further, the present invention is capable of providing the device for determining providing digital therapeutics by additionally extracting a brain activation area with respect to fNIRS data of the user who performed the mission, determining a brain state based on the additionally obtained brain activation area, and then determining and providing information related to amelioration of the brain state of the user.

Further, the present invention is capable of providing the user with an AI based brain analysis technique which allows prediction of an asymptomatic disease in advance and early diagnosis.

Further, the present invention is capable of providing the user with a brain function measurement technique which measures functions of the brain noninvasively in a way of mapping an average of time series blocks of the signal of channel position dependent fNIRS on a head, based on brain activation data imaged following measuring the brain in a state of activity.

Further, the present invention is capable of providing the user with a brain activation area, a state cognitive algorithm and a data analysis visualization technique.

Further, the present invention is capable of providing the user with a stimulation technique for obtaining brain activation data.

Further, the present invention is capable of providing the user with a Hyper-scanning technique which measures changes in the brain of several persons and Inter-brain synchrony.

Further, the present invention is capable of providing a user with a determination technique of a brain activation state.

Further, the present invention is capable of providing the user with a determination technique of the brain activation state using body information (e.g., genetic information, gait pattern information, stress information, EGG change information, a sleep state and attention change information, oxygen saturation change information, and the like). Further, the present invention aims to provide a user with a determination technique of a brain activation state using imaging scan information (e.g., MRI, PET, CT, fMRI, X-ray and the like).

Further, the present invention is capable of providing the user with a technique for predicting, diagnosing and treating a disease, based on a correlation of a plurality of data.

Further, the present invention is capable of providing the user with a technique for obtaining data capable of used, as a biomarker in real time and analyzing the same.

Further the present invention is capable of providing the user with a technique for preventing problems related to dementia, MCI, Parkinson's disease, depression, cerebral stroke, Epilepsy, brain cancer and developmental disabilities, managing, diagnosing and treating those diseases.

Further, the present invention is capable of providing the user a technique for providing feedback to the user following measuring brain functions, based on a metaverse environment.

Meanwhile, advantageous effects obtained from the present invention are not limited to the aforementioned effects, and other not-mentioned effects will be obviously understood by those skilled in the art from the description below.

Implementation may be through hardware, firmware, software or a combination of the preceding.

In a case of implementation through the hardware, methods may be implemented by one or more of ASICs (Application Specific Integrated Circuits), DSPs (Digital Signal Processors), DSPDs (Digital Signal Processing Devices), PLDs (Programmable Logic Devices), FPGAs (Field Programmable Gate Arrays), a processor, a controller, a microcontroller, a microprocessor and the like.

In a case of implementation through the firmware or the software, methods may be implemented into a form of a module, a process, a function or the like which performs the aforementioned function or operations. Source code for software is stored in a memory unit and driven by a processor. The memory unit is located on the exterior or interior of the processor, allowing receiving or transferring data from or to the processor may receive and transfer by publicly known various ways.

As aforementioned, those skilled in the art may implement the present invention referring to the detailed description of the embodiments.

The present invention may be implemented into other particular forms within the scope. Thus, the aforementioned detailed description should be considered as an example rather than understood limitedly. The scope of the present invention is determined by the claims attached hereto.

## Claims

1. A digital content-based device for providing therapeutics information, the device comprising a brain signal measurement portion (10), a brain signal stimulating portion (20), a diagnosing portion (50) including a management portion (60), and a plurality of light sources each with a corresponding detecting portion, wherein:
the brain signal stimulating portion (20) is configured to perform stimulation on a brain of a user by emitting light to a scalp of the user through the plurality of light sources, so as to obtain functional near-infrared spectroscopy, fNIRS, data of the user from the scalp to which the light was emitted through the detecting portion corresponding to each of the plurality of light sources, the fNIRS data representing a change of NIR light intensity transmitted through the scalp, wherein the device further comprises a filter for removing artifact from the fNIRS data;
the brain signal measurement portion (10) is configured to extract a first brain activation area from a plurality of brain areas of the user using the obtained fNIRS data, and the device is further configured to collect, prior to the stimulation, body information and medical imaging information both related to the user, the body information including at least one of a degree of hearing loss of the user, gait pattern of the user, a stress level, an EGG change, a sleep state and attention change information, oxygen saturation change and the like, and the medical imaging information including at least one of an MRI imaging related to the user, a CT imaging related to the user and a fMRI imaging related to the user;
the diagnosing portion (50) is configured to determine a first brain state of the user, based on information of the first brain activation area together with the body information related to the user and the medical imaging information related to the user, and by using a correlation of activation information related to each of the plurality of brain areas, the body information and the medical imaging information, based on a pre-constructed database, and by using a power spectrum method and a z-score analysis method;
the management portion (60) is configured to provide the user with a content determined corresponding to the first brain state determined by the diagnosing portion (50) under an Extended Reality, XR, environment including at least one of Augmented Reality, AR, Virtual Reality, VR, and Mixed Reality, MR;
the brain signal measurement portion (10) is further configured to extract, when the user performs a mission corresponding to the content provided by the management portion (60), a second brain activation area from the plurality of brain areas with reference to the fNIRS data of the user following performing the mission; and
the diagnosing portion (50) is further configured to determine a second brain state of the user, based on the second brain activation area, and to determine information related to amelioration of the brain state of the user by using information of the first brain state and information of the second brain state.

2. The digital content-based device for providing therapeutics information according to claim 1, wherein the management portion (60) is configured to:
determine the first brain state as a base line;
determine the content for activating the first brain activation area, corresponding to the first brain state; and
provide the user with the content through a device which the user wears, allowing the user to experience the XR environment.

3. The digital content-based device for providing therapeutics information according to claim 2, wherein the brain signal measurement portion (10) is configured to extract the second brain activation area when:
the user performs the mission changeable depending on a play processing level of the content; and
information related to the brain of the user is provided in real time while the user is in a state of performing the mission.

4. The digital content-based device for providing therapeutics information according to claim 2, wherein the management portion (60) is configured to provide the content including a relaxation content for stabilizing emotion, a problem-solving content for controlling a stressed circumstance, an engagement content, a self-control content for controlling emotion and an impulse control content for controlling an impulsive behavior.

5. The digital content-based device for providing therapeutics information according to claim 4, wherein
the user is plural in number, and
the management portion (60) is configured to provide the content including a group therapy in which at least a part of the plurality of users performs a mission together.

6. The digital content-based device for providing therapeutics information according to claim 5, wherein the diagnosing portion (50) is configured to determine a brain disease of the user based on the first brain state, and
the brain disease includes dementia, MCI, Parkinson's disease, depression, cerebral stroke, Epilepsy, brain cancer and developmental disabilities.

7. The digital content-based device for providing therapeutics information according to claim 6, wherein the management portion (60) is configured to provide the content determined relating to treatment of the determined brain disease,
the diagnosing portion (50) is configured to extract a third brain activation area from the plurality of brain areas with reference to fNIRS data of the user while performing the mission and determining a third brain state of the user based on the third brain activation area, and to
determine information related to amelioration of the brain state of the user using information of the first brain state together with information of the second brain state and information of the third brain state.

8. The digital content-based device for providing therapeutics information according to claim 1, further configured to provide the user with at least one of information of the first brain state, information of the second brain state and amelioration information of the brain state.

9. The digital content-based method for providing therapeutics information according to claim 1, wherein the diagnosing portion (50) is configured to calculate analysis data for a supplementary Motor Area, SMA, which controls the user's movement based on the first brain activation area to determine the first brain state based of the calculated analysis data.

10. The digital content-based device for providing therapeutics information according to claim 9, wherein the diagnosing portion (50) is configured to determine, in the situation that a specific event happened to the user, that there is a limit in movement of the target object as an analysis data value for the SMA is increased with reference to a reference line, whereas it is determined that the target object is adept in movement as the analysis data value is decreased with reference to a reference line.

## Patentansprüche

1. Eine auf digitalem Inhalt basierende Vorrichtung zum Bereitstellen von therapeutischen Informationen, wobei die Vorrichtung einen Hirnsignalmessabschnitt (10), einen Hirnsignalstimulationsabschnitt (20), einen Diagnoseabschnitt (50) mit einem Verwaltungsabschnitt (60) und eine Vielzahl von Lichtquellen mit jeweils einem entsprechenden Erfassungsabschnitt umfasst, wobei:
der Hirnsignalstimulationsabschnitt (20) konfiguriert ist, um eine Stimulation an einem Gehirn eines Benutzers durchzuführen, indem Licht an eine Kopfhaut des Benutzers durch die Vielzahl von Lichtquellen emittiert wird, um funktionelle Daten von Nahinfrarotspektroskopie, fNIRS (*functional near-infrared spectroscopy*), des Benutzers von der Kopfhaut zu erhalten, an die das Licht durch den Erfassungsabschnitt emittiert wurde, entsprechend jeder der Vielzahl von Lichtquellen, wobei die fNIRS-Daten eine Änderung der NIR-Lichtintensität darstellen, die durch die Kopfhaut übertragen wird, wobei die Vorrichtung ferner einen Filter zum Entfernen von Artefakt aus den fNIRS-Daten umfasst;
der Hirnsignalmessabschnitt (10) dazu konfiguriert ist, einen ersten Hirnaktivierungsbereich aus einer Vielzahl von Hirnbereichen des Benutzers unter Verwendung der erhaltenen fNIRS-Daten zu extrahieren, und die Vorrichtung ferner dazu konfiguriert ist, vor der Stimulation Körperinformationen und medizinische Bildgebungsinformationen, die sich beide auf den Benutzer beziehen, zu sammeln, wobei die Körperinformationen mindestens eines von einem Grad an Hörverlust des Benutzers, einem Gangmuster des Benutzers, einem Stressniveau, einer EGG-Änderung, einem Schlafzustand und Aufmerksamkeitsänderungsinformationen, einer Sauerstoffsättigungsänderung und dergleichen beinhalten, und die medizinischen Bildgebungsinformationen mindestens eines von einer MRT-Bildgebung, die sich auf den Benutzer bezieht, einer CT-Bildgebung, die sich auf den Benutzer bezieht, und einer fMRT-Bildgebung, die sich auf den Benutzer bezieht, beinhalten;
der Diagnoseabschnitt (50) dazu konfiguriert ist, einen ersten Hirnzustand des Benutzers auf der Grundlage von Informationen des ersten Hirnaktivierungsbereichs zusammen mit den Körperinformationen, die sich auf den Benutzer beziehen, und den medizinischen Bildgebungsinformationen, die sich auf den Benutzer beziehen, und unter Verwendung von einer Korrelation von Aktivierungsinformationen, die sich auf jeden von der Vielzahl von Hirnbereichen, den Körperinformationen und den medizinischen Bildgebungsinformationen beziehen, auf der Grundlage von einer vorkonstruierten Datenbank und unter Verwendung von einem Leistungsspektrumverfahren und einem Z-Score-Analyseverfahren zu bestimmen;
der Verwaltungsabschnitt (60) dazu konfiguriert ist, dem Benutzer einen Inhalt bereitzustellen, der entsprechend dem ersten Hirnzustand bestimmt worden ist, der durch den Diagnoseabschnitt (50) unter einer Umgebung von Extended Reality, XR, bestimmt wird, welche mindestens eines von Augmented Reality, AR, Virtual Reality, VR und Mixed Reality, MR umfasst;
der Hirnsignalmessabschnitt (10) ferner dazu konfiguriert ist, wenn der Benutzer eine Aufgabe durchführt, die dem durch den Verwaltungsabschnitt (60) bereitgestellten Inhalt entspricht, einen zweiten Hirnaktivierungsbereich aus der Vielzahl von Hirnbereichen unter Bezugnahme auf die fNIRS-Daten des Benutzers nach Durchführung der Aufgabe zu extrahieren; und
der Diagnoseabschnitt (50) ferner dazu konfiguriert ist, einen zweiten Hirnzustand des Benutzers auf Grundlage des zweiten Hirnaktivierungsbereichs zu bestimmen und Informationen in Bezug auf eine Verbesserung des Hirnzustands des Benutzers unter Verwendung von Informationen des ersten Hirnzustands und Informationen des zweiten Hirnzustands zu bestimmen.

2. Die auf digitalem Inhalt basierende Vorrichtung zum Bereitstellen von therapeutischen Informationen nach Anspruch 1, wobei der Verwaltungsabschnitt (60) dazu konfiguriert ist:
den ersten Hirnzustands als Basislinie zu bestimmen;
den Inhalt zum Aktivieren des ersten Hirnaktivierungsbereichs, der dem ersten Hirnzustand entspricht zu bestimmen; und
dem Benutzer den Inhalt über ein Gerät zur Verfügung zu stellen, das der Benutzer trägt, damit der Benutzer die XR-Umgebung erleben kann.

3. Die auf digitalem Inhalt basierende Vorrichtung zum Bereitstellen von therapeutischen Informationen nach Anspruch 2, wobei der Hirnsignalmessabschnitt (10) dazu konfiguriert ist, den zweiten Hirnaktivierungsbereich zu extrahieren, wenn:
der Benutzer die Aufgabe änderbar in Abhängigkeit von einer Wiedergabeverarbeitungsebene des Inhalts durchführt; und
Informationen in Bezug auf das Gehirn des Benutzers in Echtzeit bereitgestellt werden, während sich der Benutzer in einem Zustand der Durchführung der Aufgabe befindet.

4. Die auf digitalem Inhalt basierende Vorrichtung zum Bereitstellen von therapeutischen Informationen nach Anspruch 2, wobei der Verwaltungsabschnitt (60) dazu konfiguriert ist, den Inhalt bereitzustellen, der einen Entspannungsinhalt zum Stabilisieren von Emotionen, einen Problemlösungsinhalt zum Steuern eines gestressten Umstands, einen Engagement-Inhalt, einen Selbststeuerungsinhalt zum Steuern von Emotionen und einen Impulssteuerungsinhalt zum Steuern eines Impulsverhaltens enthält.

5. Die auf digitalem Inhalt basierende Vorrichtung zum Bereitstellen von therapeutischen Informationen nach Anspruch 4, wobei
der Benutzer in eine Pluralzahl existiert, und
der Verwaltungsabschnitt (60) konfiguriert ist, um den Inhalt bereitzustellen, der eine Gruppentherapie beinhaltet, bei der mindestens ein Teil der Vielzahl von Benutzern eine Aufgabe zusammen durchführt.

6. Die auf digitalem Inhalt basierende Vorrichtung zum Bereitstellen von therapeutischen Informationen nach Anspruch 5, wobei der Diagnoseabschnitt (50) dazu konfiguriert ist, eine Gehirnerkrankung des Benutzers auf der Grundlage des ersten Hirnzustands zu bestimmen, und
die Gehirnerkrankung Demenz, LKB, Parkinson-Krankheit, Depression, Schlaganfall, Epilepsie, Hirntumor und Entwicklungsstörungen umfasst.

7. Die auf digitalem Inhalt basierende Vorrichtung zum Bereitstellen von therapeutischen Informationen nach Anspruch 6, wobei der Verwaltungsabschnitt (60) dazu konfiguriert ist, den Inhalt bereitzustellen, der in Bezug auf die Behandlung der bestimmten Gehirnerkrankung bestimmt wurde,
der Diagnoseabschnitt (50) dazu konfiguriert ist, einen dritten Hirnaktivierungsbereich aus der Vielzahl von Hirnbereichen unter Bezugnahme auf fNIRS-Daten des Benutzers zu extrahieren, während die Aufgabe durchgeführt wird und ein dritter Hirnzustand des Benutzers auf Grundlage des dritten Hirnaktivierungsbereichs bestimmt wird, und
Informationen im Zusammenhang mit der Verbesserung des Hirnzustands des Benutzers unter Verwendung von Informationen des ersten Hirnzustands zusammen mit Informationen des zweiten Hirnzustands und Informationen des dritten Hirnzustands zu bestimmen.

8. Die auf digitalem Inhalt basierende Vorrichtung zum Bereitstellen von therapeutischen Informationen nach Anspruch 1, die ferner dazu konfiguriert ist, dem Benutzer eine von Informationen des ersten Hirnzustands, Informationen des zweiten Hirnzustands und Verbesserungsinformationen des Hirnzustands bereitzustellen.

9. Das auf digitalem Inhalt basierende Verfahren zum Bereitstellen von therapeutischen Informationen nach Anspruch 1, wobei der Diagnoseabschnitt (50) dazu konfiguriert ist, Analysedaten für einen supplementärmotorischen Cortex, SMA (*supplementary motor area*), zu berechnen, der die Bewegung des Benutzers basierend auf dem ersten Hirnaktivierungsbereich steuert, um den ersten Hirnzustand basierend auf den berechneten Analysedaten zu bestimmen.

10. Die auf digitalem Inhalt basierende Vorrichtung zum Bereitstellen von therapeutischen Informationen nach Anspruch 9, wobei der Diagnoseabschnitt (50) dazu konfiguriert ist, in der Situation, dass ein spezifisches Ereignis dem Benutzer passiert ist, zu bestimmen, dass es eine Begrenzung in der Bewegung des Zielobjekts gibt, wenn ein Analysedatenwert für den SMA mit Bezug auf eine Referenzlinie erhöht wird, während bestimmt wird, dass das Zielobjekt in der Bewegung versiert ist, wenn der Analysedatenwert mit Bezug auf eine Referenzlinie verringert wird.

## Revendications

1. Un dispositif basé sur du contenu numérique pour fournir des informations thérapeutiques, le dispositif comprenant une partie de mesure de signal de cerveau (10), une partie de stimulation de signal du cerveau (20), une partie de diagnostic (50) comprenant une partie de gestion (60), et une pluralité de sources de lumière chacune avec une partie de détection correspondante, dans lequel :
la partie de stimulation de signal du cerveau (20) est configurée pour effectuer une stimulation sur un cerveau d'un utilisateur en émettant de la lumière vers un cuir chevelu de l'utilisateur à travers la pluralité de sources de lumière, de manière à obtenir des données d'imagerie spectroscopique proche infrarouge fonctionnelle, fNIRS (*functional near-infrared spectroscopy*), de l'utilisateur à partir du cuir chevelu vers lequel la lumière a été émise à travers la partie de détection correspondant à chacune de la pluralité de sources de lumière, les données de fNIRS représentant un changement d'intensité de lumière NIR transmise à travers le cuir chevelu, dans lequel le dispositif comprend en outre un filtre pour éliminer des artefacts des données de fNIRS ;
la partie de mesure de signal de cerveau (10) est configurée pour extraire une première zone d'activation cérébrale à partir d'une pluralité de zones de cerveau de l'utilisateur en utilisant les données de fNIRS obtenues, et le dispositif est en outre configuré pour collecter, avant la stimulation, des informations du corps et des informations d'imagerie médicale toutes deux liées à l'utilisateur, les informations du corps comprenant au moins un d'un degré de perte auditive de l'utilisateur, d'un modèle de démarche de l'utilisateur, d'un niveau de stress, d'un changement d'EGG, d'un état de sommeil et d'informations de changement d'attention, d'un changement de saturation d'oxygène et similaires, et les informations d'imagerie médicale comprenant au moins une parmi une imagerie d'IRM liée à l'utilisateur, une imagerie de tomographie par ordinateur liée à l'utilisateur et une imagerie de IRMf liée à l'utilisateur ;
la partie de diagnostic (50) est configurée pour déterminer un premier état du cerveau de l'utilisateur, sur la base d'informations de la première zone d'activation cérébrale conjointement avec les informations du corps relatives à l'utilisateur et les informations d'imagerie médicale relatives à l'utilisateur, et en utilisant une corrélation d'informations d'activation relatives à chacune de la pluralité de zones cérébrales, les informations du corps et les informations d'imagerie médicale, sur la base d'une base de données préconstruite, et en utilisant un procédé de spectre de puissance et un procédé d'analyse de scores z ;
la partie de gestion (60) est configurée pour fournir à l'utilisateur un contenu déterminé correspondant au premier état du cerveau déterminé par la partie de diagnostic (50) dans un environnement de réalité étendue, XR, comprenant au moins l'un parmi la réalité augmentée, AR, la réalité virtuelle, VR et la réalité mixte, MR ;
la partie de mesure de signal de cerveau (10) est en outre configurée pour extraire, lorsque l'utilisateur effectue une mission correspondant au contenu fourni par la partie de gestion (60), une seconde zone d'activation cérébrale de la pluralité de zones cérébrales en référence aux données de fNIRS de l'utilisateur après l'exécution de la mission ; et
la partie de diagnostic (50) est en outre configurée pour déterminer un second état du cerveau de l'utilisateur, sur la base de la seconde zone d'activation cérébrale, et pour déterminer des informations liées à l'amélioration de l'état du cerveau de l'utilisateur en utilisant des informations du premier état du cerveau et des informations du second état du cerveau.

2. Le dispositif basé sur du contenu numérique pour fournir des informations thérapeutiques selon la revendication 1, dans lequel la partie de gestion (60) est configurée pour :
déterminer le premier état du cerveau en tant que ligne de base ;
déterminer le contenu pour activer la première zone d'activation cérébrale, correspondant au premier état du cerveau ; et
fournir à l'utilisateur le contenu à travers un dispositif que l'utilisateur porte, permettant à l'utilisateur de faire l'expérience de l'environnement XR.

3. Le dispositif basé sur du contenu numérique pour fournir des informations thérapeutiques selon la revendication 2, dans lequel la partie de mesure de signal de cerveau (10) est configurée pour extraire la seconde zone d'activation cérébrale lorsque :
l'utilisateur effectue la mission modifiable en fonction d'un niveau de traitement de lecture du contenu ; et
des informations relatives au cerveau de l'utilisateur sont fournies en temps réel alors que l'utilisateur est dans un état d'exécution de la mission.

4. Le dispositif basé sur du contenu numérique pour fournir des informations thérapeutiques selon la revendication 2, dans lequel la partie de gestion (60) est configurée pour fournir le contenu comprenant un contenu de relaxation pour stabiliser l'émotion, un contenu de résolution de problème pour contrôler une circonstance de stress, un contenu d'engagement, un contenu d'auto-contrôle pour contrôler l'émotion et un contenu de contrôle d'impulsions pour contrôler un comportement impulsif.

5. Le dispositif basé sur du contenu numérique pour fournir des informations thérapeutiques selon la revendication 4, dans lequel
l'utilisateur est au nombre de plusieurs, et
la partie de gestion (60) est configurée pour fournir le contenu comprenant une thérapie de groupe dans laquelle au moins une partie de la pluralité d'utilisateurs effectue une mission ensemble.

6. Le dispositif basé sur du contenu numérique pour fournir des informations thérapeutiques selon la revendication 5, dans lequel la partie de diagnostic (50) est configurée pour déterminer une maladie cérébrale de l'utilisateur sur la base du premier état du cerveau, et
la maladie cérébrale comprend la démence, le DCL, la maladie de Parkinson, la dépression, un accident vasculaire cérébral, l'épilepsie, le cancer du cerveau et les troubles du développement.

7. Le dispositif basé sur du contenu numérique pour fournir des informations thérapeutiques selon la revendication 6, dans lequel la partie de gestion (60) est configurée pour fournir le contenu déterminé concernant le traitement de la maladie cérébrale déterminée,
la partie de diagnostic (50) est configurée pour extraire une troisième zone d'activation cérébrale de la pluralité de zones cérébrales en référence aux données fNIRS de l'utilisateur tout en effectuant la mission et en déterminant un troisième état du cerveau de l'utilisateur sur la base de la troisième zone d'activation cérébrale, et pour
déterminer des informations liées à l'amélioration de l'état du cerveau de l'utilisateur en utilisant des informations du premier état du cerveau avec des informations du second état du cerveau et des informations du troisième état du cerveau.

8. Le dispositif basé sur du contenu numérique pour fournir des informations thérapeutiques selon la revendication 1, configuré en outre pour fournir à l'utilisateur au moins l'une des informations du premier état du cerveau, des informations du second état du cerveau et des informations d'amélioration de l'état du cerveau.

9. Le procédé basé sur du contenu numérique pour fournir des informations thérapeutiques selon la revendication 1, dans lequel la partie de diagnostic (50) est configurée pour calculer des données d'analyse pour une aire motrice supplémentaire, AMS, qui commande le mouvement de l'utilisateur sur la base de la première zone d'activation de cerveau pour déterminer le premier état du cerveau sur la base des données d'analyse calculées.

10. Le dispositif basé sur du contenu numérique pour fournir des informations thérapeutiques selon la revendication 9, dans lequel la partie de diagnostic (50) est configurée pour déterminer, dans la situation où un événement spécifique est arrivé à l'utilisateur, qu'il y a une limite de mouvement de l'objet cible lorsqu'une valeur de données d'analyse pour l'AMS est augmentée par rapport à une ligne de référence, alors qu'il est déterminé que l'objet cible est apte au mouvement lorsque la valeur de données d'analyse est diminuée par rapport à une ligne de référence.
